(19) Europäisches Patentamt
European Patent Office
Office européen des brevets

(11) Publication number: **0 080 115**
**B1**

(12) # EUROPEAN PATENT SPECIFICATION

(45) Date of publication of patent specification: **13.05.87**

(51) Int. Cl.$^4$: **C 07 D 265/34,** A 61 K 31/535

(21) Application number: **82110409.8**

(22) Date of filing: **11.11.82**

(54) **Hexahydronaphth(1,2-b)-1,4-oxazines, process for their preparation and pharmaceutical formulation containing them.**

(30) Priority: **20.11.81 US 323349**

(43) Date of publication of application:
**01.06.83 Bulletin 83/22**

(45) Publication of the grant of the patent:
**13.05.87 Bulletin 87/20**

(84) Designated Contracting States:
**AT BE CH DE FR GB IT LI LU NL SE**

(56) References cited:
**US-A-3 642 789**
**US-A-4 238 486**

**Chemical Abstracts vol. 69, no. 10, 2 September 1968, Columbus, Ohio, USA A.I. KIPRIANOV et al. "Cyanine dyes from 1,4-benzoxazine and 1,4-benzoselenazine derivatives", page 3480, column 1, abstract no. 37081c**

**Chemical Abstracts vol. 82, no. 7, 17 February 1975, Columbus, Ohio, USA R.T. COUTTS et al. "Synthesis and properties of 4-acetoxy-2,3-dihydro-3-oxo-4H-naphth1,2-br1,4roxazine, an analog of known carcinogenic hydroxamic acids", page 430, column 1, abstract no. 43293h**

(73) Proprietor: **MERCK & CO. INC.**
**126, East Lincoln Avenue P.O. Box 2000**
**Rahway New Jersey 07065 (US)**

(72) Inventor: **Jones, James H.**
**649 Midway Lane**
**Blue Bell, PA. 19446 (US)**
Inventor: **McClure David E.**
**1402 St. Andrews Way**
**Lansdale, PA. 19446 (US)**
Inventor: **Grenda, Victor J.**
**15 Loriann Road**
**Warren, NJ. 07060 (US)**

(74) Representative: **Blum, Rudolf Emil Ernst et al**
**c/o E. Blum & Co Patentanwälte Vorderberg 11**
**CH-8044 Zürich (CH)**

(56) References cited:
**Chemical Abstracts vol. 76-85, 9th collective index, 1972-1976. Chemical substances. Molybdenum oxide - nitroemal, Columbus, Ohio, USA page 24436CS**

**EP 0 080 115 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Background of the Invention

The tetracyclic ergoline type of compound (US—A—3,968,111) has pharmaceutical activity, and the indolobenzoazines (US—A—4,238,486) in which the D-ring of the ergolines is a tetrahydrooxazines have antihypertensive and antiparkinson activity.

Summary of the Invention

This invention is concerned with *trans*-1a,2,3,4a,5,6-hexyhydronaphth[1,2-b]-1,4-oxazine and derivatives of structural formula I:

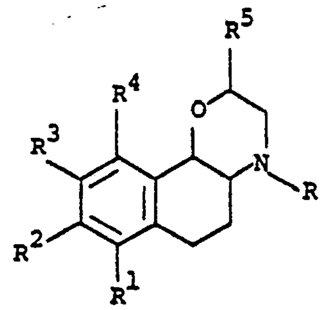

I

and pharmaceutically acceptable salts thereof. In the inventive compounds, accordingly, the hydro-oxazine nucleus is condensed with the nonaromatic nucleus of the tetrahydro-naphthalene.

The novel compounds of formula I and their salts demonstrate the dopaminergic activity of antiparkinson and antihypertensive agents. Some of the novel compounds are also $\alpha_2$-adrenergic receptor antagonists and hence antidepressant.

Description of the Prior Art

In Chemical Abstracts vol. 76—85, 9th collective index, 1972-1976 there are described 2H-naphth[1,2-b]-1,4-oxazines. In said compounds, accordingly, the oxazine nucleus possesses a double bond and the naphthalene ring which is condensed with it is not hydrogenated.

In Chemical Abstracts vol. 69, no. 10, 2 September 1968 there are disclosed 1,4 oxazine derivatives in which as well a not hydrogenated naphthalene nucleus is condensed with the oxazine nucleus and in which furthermore one carbon atom of the oxazine nucleus is a carbonyl group.

In the US—A—3 642 789 there are disclosed indeno-1,4-oxazine compounds, in which, accordingly, with the oxazine nucleus there is condensed a 5-membered hydrogenated nucleus of a corresponding indeno structure.

In the US—A—4 238 486 there are described compounds which have totally four condensed rings and not only three condensed rings as the claimed compounds. In said compounds a further nitrogen containing heterocyclic ring is condensed to the not aromatic ring of a tetrahydro-naphthalene structure. Said compounds have a similar pharmaceutical activity as the inventive compounds.

In Chemical Abstracts vol. 82, no. 7, 17 February 1975 there are however described compounds which are structurally related to the inventive compounds of formula I. Said compounds, however, have a ketonic group in their oxazine nucleus and they are analogs of known carcinogenic substances. They, accordingly, differ in their pharmaceutical activity from the inventive compounds.

A further object of the present invention are pharmaceutical compositions for the treatment of Parkinsonism, hypertension and depression which contain the inventive compounds or pharmaceutically acceptable salts thereof as pharmaceutically active ingredient.

Detailed Description of the Invention

One subject of the present invention are new compounds of structural formula I

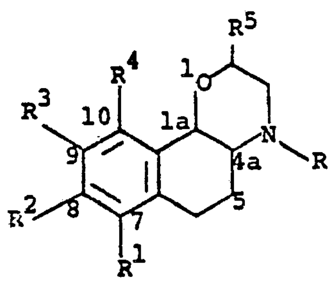

(trans) I

with positive, negative or zero optical activity, or salts thereof, wherein:

R is

a) hydrogen,
b) $C_{1-4}$ alkyl,
c) $C_{2-5}$ alkenyl,
d) phenyl-$C_{1-4}$ alkyl; and

$R^1$, $R^2$, $R^3$ and $R^4$ are independently:

a) hydrogen,
b) $C_{1-4}$ alkyl,
c) halo,
d) $OR^6$ wherein $R^6$ is
  1) hydrogen,
  2) $C_{1-3}$ alkyl,
  3) phenyl-$C_{1-3}$ alkyl,
  4) $-\underset{\underset{O}{\|}}{C}-R^7$ wherein $R^7$ is
    i) $C_{1-6}$ alkyl
    ii) $C_{3-6}$ cycloalkyl
    iii) phenyl-$C_{1-3}$ alkyl, wherein the phenyl group is unsubstituted or substituted with one or more halo, $C_{1-3}$ alkyl, or $C_{1-3}$ alkoxy
    iv) pyridyl-$C_{1-3}$ alkyl or furyl-$C_{1-3}$ alkyl,
    v) phenyl, either unsubstituted or substituted with one or more halo, $C_{1-3}$ alkyl, or $C_{1-3}$ alkoxy,
    vi) pyridyl or furyl and
    vii) $-NR^8R^9$, wherein $R^8$ and $R^9$ are independently hydrogen or $C_{1-3}$ alkyl, or $R^8$ and $R^9$ may be joined together to form a heterocyclic piperidinyl, morpholinyl, piperazinyl or N—$C_{1-3}$ alkylpiperazinyl ring with the nitrogen to which they are attached,

or

e) two adjacent groups selected from $R^1$, $R^2$, $R^3$ and $R^4$ form together an alkylene-dioxy group

and

$R^5$ is hydrogen, $C_{1-3}$ alkyl or phenyl.

The structural formula I stated above is used herein to represent the trans-isomer only.

In preferred compounds of formula I R and $R^5$ have the meaning stated above and $R^1$, $R^2$, $R^3$ and $R^4$ are independently:

a) hydrogen,
b) $C_{1-4}$ alkyl,
c) halo,
d) $OR^6$

wherein $R^6$ is

  1) hydrogen,
  2) $C_{1-3}$ alkyl,
  3) phenyl-$C_{1-3}$ alkyl,
  4) $-\underset{\underset{O}{\|}}{C}-R^7$

wherein $R^7$ is as defined above in i) through vi) or

    vii) $-NR^8R^9$, wherein $R^8$ and $R^9$ are independently hydrogen or $C_{1-3}$ alkyl, or $R^8$ and $R^9$ may be joined together to form with the nitrogen to which they are attached a heterocycle selected from piperidinyl, morpholinyl, piperazinyl and N—$C_{1-3}$ alkylpiperazinyl

or

e) two adjacent groups selected from $R^1$, $R^2$, $R^3$ and $R^4$ form together a methylene-dioxy group.

In the inventive compounds of formula I, R can be a branched or straight chain alkyl having 1—4 carbon atoms, preferably ethyl or propyl, an alkenyl group having 2—5 carbon atoms, preferably allyl, or a phenyl substituted alkyl group having 1—4 carbon atoms in the alkyl moiety, preferably benzyl.

In the inventive compounds of formula I the radicals $R^1$, $R^2$, $R^3$ and $R^4$ are selected from the group comprising hydrogen, alkyl groups having 1—4 carbon atoms, preferably methyl groups, halo, preferably fluoro, chloro or bromo or groups of formula $-OR^6$ wherein $R^6$ is hydrogen, an alkyl group having 1—3 carbon atoms, preferably methyl, a phenyl alkyl group having 1—3 carbon atoms in the alkyl part, preferably benzyl or an acyl group of formula

$$-\overset{\overset{O}{\|}}{C}-R^7$$

In said acyl group $R^7$ is selected from straight or branched chain alkyl groups having 1—6 carbon atoms, preferably methyl or t-butyl, cycloalkyl groups having 3—6 carbon atoms, preferably cyclopropyl or cyclohexyl, phenyl-alkyl groups having 1—3 carbon atoms in the alkyl group, wherein the phenyl group is unsubstituted or substituted with one or more halo atoms or alkyl or alkoxy groups having 1—3 carbon atoms or $R^7$ has the meaning stated above in iv) through vii).

In preferred compounds of formula I, furthermore, $R^5$ is hydrogen and they, accordingly, have the structural formula Ia

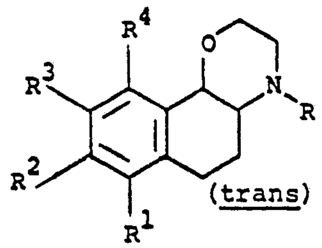

Ia

wherein:

R has the meaning stated above and

$R^1$, $R^2$, $R^3$ and $R^4$ are independently:

- a) hydrogen,
- b) $C_{1-4}$ alkyl,
- c) halo,
- d) $OR^6$ wherein $R^6$ is
  - 1) hydrogen
  - 2) $C_{2-3}$ alkyl
  - 3) phenyl-$C_{1-3}$ alkyl,
  - 4) $-\underset{\underset{O}{\|}}{C}-R^7$

$R^2$, $R^3$ and $R^4$ form together a methylenedioxy group.

In preferred compounds of formula Ia, R is an alkyl group having 1—4 carbon atoms, preferably an ethyl group or a n-propyl group and the radicals $R^1$, $R^2$, $R^3$ and $R^4$ are hydrogen, hydroxy, methoxy, acetoxy or pivaloyloxy specially in the 9-position.

In preferred compounds of formula Ia stated above, furthermore, R is n-propyl, $R^3$ is hydroxy or methoxy and $R^1$, $R^2$ and $R^4$ are hydrogen. Said compounds, accordingly, have the structural formula

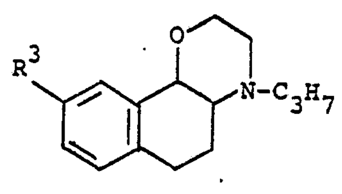

wherein $R^3$ is $-OH$ or $-OCH_3$.

The novel compounds of formula I have two asymmetric carbon atoms, 4a and 1a, where the morpholino ring is fused to the tetrahydronaphthalene ring. This invention includes stereoisomers in which these asymmetric centers are in a *trans* conformation. The invention further includes the individual enantiomers and mixtures thereof including the racemate.

Both enantiomers and mixtures thereof have dopaminergic activity, but the (+)-*trans* compound is much preferred for that utility. On the other hand, the (−)-*trans* enantiomer is preferred for $\alpha_2$-adrenergic receptor blockade.

The pure optical antipodes can be prepared from the appropriate optically pure intermediates or by resolution of the final racemic product.

The pharmaceutically acceptable salts of the novel compound of this invention are acid addition salts formed from a novel compound and an organic or inorganic acid recognized by the art as providing a pharmaceutically acceptable acid addition salt, such as hydrochloride, hydrobromide, dihydrogen phosphate, sulfate, citrate, pamoate, pyruvate, napsylate, isethionate, maleate, fumarate, or the like.

These salts are readily prepared by mixing solutions of equimolecular amounts of the free base compound and the desired acid in suitable solvents such as water, alcohols, ether or chloroform, followed by recovery of the product by collecting the precipitated salt or evaporation of the solvent.

A novel process for preparing the novel compounds is another embodiment of this invention and is represented as follows:

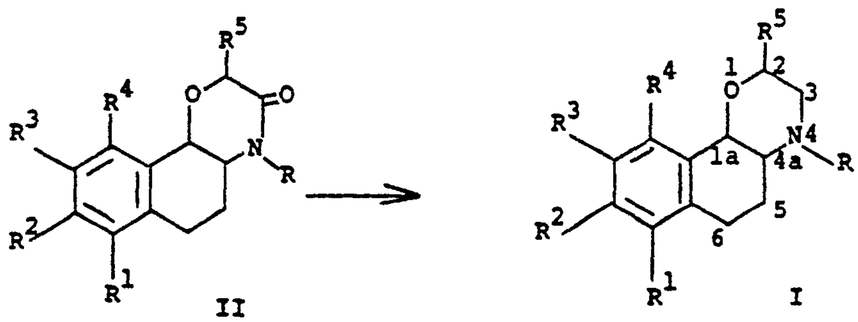

II

I

The novel process comprises the reduction of the oxazinone, II, with a complex metal hydride such as lithium aluminum hydride in an inert organic solvent such as an ether, for example, tetrahydrofuran, 1,2-dimethoxyethane, tetrahydropyran, or the like at a temperature between about 0°C and 100°C. Operating procedures normally involve slow addition of the cyclic amide to the reducing agent at room temperature or below, followed by heating to an elevated temperature within the stated range, preferably, the reflux temperature of the solvent, for about 1/2 to about 8 hours usually about 4 hours.

If, in the novel process of this invention, the nitrogen substituent, R, is hydrogen, and it is desired that it be one of the other substituents within the definition of R the product from the foregoing reaction may be treated with the appropriate alkylating agent of formula:

$$R—X$$

wherein R is as defined earlier and X is a suitable leaving group such as iodo, bromo, chloro, mesylate, tosylate, or the like. The alkylation is conducted in an organic solvent such as DMF, DMSO, or THF, preferably DMF, at about 10 to about 100°C until the reaction is substantially complete; usually about 3 to 10 hours.

If one or more of $R^1$, $R^3$ or $R^4$ is alkoxy, it or they may be converted to hydroxy, if desired, by heating at about 150—250°C in excess pyridine hydrochloride for about 3 to 8 hours. Alternatively, the deetherification can be accomplished by treatment with boron tribromide ($BBr_3$) or aluminum chloride ($AlCl_3$) in an inert organic solvent such as petroleum ether, or hexane, or a chlorinated hydrocarbon such as methylene chloride, tetrachloroethane or the like, between room temperature and reflux temperature for 3 to about 8 hours.

Also, if $R^1$, $R^2$, $R^3$ or $R^4$ is benzyloxy or substituted benzyloxy it or they can be converted to hydroxy by hydrogenolysis with a noble metal catalyst such as palladium, platinum, or platinum oxide, with or without a carrier such as carbon, or the like, preferably palladium on carbon, in an inert organic solvent such as $C_{1-3}$ alkanol, an ethereal solvent such as tetrahydrofuran, 1,2-dimethoxyethane, or the like, or mixtures thereof. The reaction is conducted at or near room temperature such as 15—30°C until hydrogen uptake ceases or the requisite amount of hydrogen is consumed.

In those compounds wherein $R^1$, $R^2$, $R^3$ or $R^4$ is

$$—O—\overset{\overset{O}{\|}}{C}—R^7$$

they are prepared by treatment of the corresponding hydroxy compound with the appropriate acid anhydride

$$R^7—\overset{\overset{O}{\|}}{C}—O—\overset{\overset{O}{\|}}{C}—R^7 \text{ acid chloride } R^7—\overset{\overset{O}{\|}}{C}—Cl\text{, or the like.}$$

in the presence of an acylation catalyst such as pyridine, 4-dimethylaminopyridine or 4-pyrolidinopyridine. Temperatures from about 15°C to 100°C are used until the reaction is substantially complete.

A third embodiment of this invention is a pharmaceutical formulation comprising one of the novel compounds as active ingredient. It may be in any art-recognized form suitable for oral use, such as tablets, troches, lozenges, aqueous or oil suspensions, dispersible powders, or granules, emulsions, hard or soft capsules, syrups, or elixirs. For intravenous, intramuscular and subcutaneous use the pharmaceutical compositions may be in any art recognized form of a sterile injectable preparation such as a sterile aqueous or oleaginous solution or suspension. The amount of active ingredient incorporated in a unit dosage of the

above described pharmaceutical compositions may be from 1 to 400 mg, and preferably from 5 to 250 mg.

Other embodiments of this invention are the treatment of hypertension and/or parkinsonism with a compound of Formula I with zero or positive optical activity. The route of administration can be oral, rectal, intravenous, intramuscular, or subcutaneous. Doses of 0.1 to 20 mg/kg/day and preferably of 0.5 to 10 mg/kg/day of active ingredient are generally adequate, and if preferred it can be administered in divided doses given two to four times daily.

In the treatment of Parkinsonism, this invention also includes the coadministration of a compound of Formula I with zero or positive optical rotation with an art-recognized antiparkinsonism agent such as SINEMET® (Merck & Co., Inc., Rahway, N.J.), each agent being administered in a dose comparable to that recommended for use as though it were the sole therapeutic agent. The coadministration is accomplished either by administration of the two agents separately at approximately the same time or by administration of a single unit dosage form comprising the combined agents such as: 10 mg of a compound of Formula I with zero or positive optical rotation; 100 mg of l-dopa; and 10 mg of carbidopa. The coadministration is particularly useful when, as frequently happens, parkinsonism symptoms break through despite a history of successful treatment with SINEMET® or other art-recognized antiparkinsonism agent.

A further method of treatment, comprising another embodiment of this invention, is the treatment of depression with a compound of Formula I with zero or negative optical activity. Doses and modes of administration comparable to those described above are satisfactory for this novel method of treatment.

It is to be noted that the precise unit dosage form and dosage level depend upon the requirements of the individual being treated, and consequently are left to the discretion of the therapist.

Example 1

*trans*-1a,2,3,4a,5,6-Hexahydro-4H-naphth[1,2-b]-1,4-oxazine hydrochloride

*Step A:* Preparation of 2-chloroacetamido-3,4-dihydronaphthalen-1(2H)-one

To a stirred solution of sodium bicarbonate (4.0 g) in water (50 ml) layered with ethyl acetate (200 ml) was added solid 2-amino-3,4-dihydronaphthalen-1(2H)-one hydrochloride (4.0 g, 2 mmol). After the solid had dissolved chloroacetylchloride (1.6 ml, 2 mmol) was added dropwise over 10 minutes. After stirring for 1 hr, the ethyl acetate layer was separated, washed with brine and dried over $Na_2SO_4$. Evaporation afforded 2.7 g (57%) of a dark solid; m.p. 118—122°C. The product was purified by chromatography on silica using ethyl acetate: hexane (1:1 v/v) to elute, m.p. 121—123°C.

*Step B:* Preparation of *trans*-2-Chloroacetamido-1,2,3,4-tetrahydronaphthalen-1-ol

To a solution of 2-chloroacetamido-3,4-dihydronaphthalen-1(2H)one (2.3 g, 1 mmole) in ethanol (50 ml) and chloroform (20 ml) was added sodium borohydride (500 mg) in portions. After 1 hr, several drops of acetic acid were added to destroy excess sodium borohydride. The mixture was poured into water (150 ml) and the product was extracted into chloroform. The chloroform was separated, dried ($Na_2SO_4$) and evaporated to yield 1.7 g (71%) of a white solid m.p. 126—130°C. After recrystallization from butylchloride it had m.p. 136—138°C.

*Step C:* Preparation of *trans*-1a,2,4,4a,5,6-Hexahydro-4H-naphth[1,2-b]-1,4-oxazin-3-one

To a suspension of NaH (2.4 g, 53% mineral oil) in DMF (24 ml) was added a solution of *trans*-2-chloroacetamido-1,2,3,4-tetrahydronaphthalen-1-ol (5.5 g, 2.3 mmole). The reaction mixture was stirred at room temperature for 3 hr, some ethanol was added to destroy excess NaH and the reaction mixture was poured into water (150 ml). The resulting solid was filtered and dried to yield 3.1 g (71%) of product, m.p. 225°C (dec). After recrystallization from butyl chloride it had m.p. 232—235°C (dec).

*Step D:* Preparation of *trans*-1a,2,3,4a,5,6,-Hexahydro-4H-naphth(1,2-b]-1,4-oxazine hydrochloride

To a stirred suspension of $LiAlH_4$ (2.0 g) in THF (100 ml) was added a solution of *trans*-1a,2,4,4a,5,6-hexahydronaphth[1,2-b]-1,4-oxazin-3-one (2.4 g, 1.2 mmole) in THF (250 ml). The reaction mixture was heated at reflux for 4 hrs, cooled in an ice bath, and ethanol was added to destroy excess $LiAlH_4$. Rochell salt (100 ml of a 20% (w/v aqueous) solution) was added and the mixture was extracted with ethyl acetate (3 × 300 ml). The ethyl acetate layer was dried ($Na_2SO_4$) treated with decolorizing carbon, filtered, and evaporated to a red oil. The oil was dissolved in 20 ml of 1:1 (v/v) acetone-ether and several ml of methanolic hydrochloric acid was added. The resulting solid was filtered and dried to yield 2.5 g of product, m.p. 290°C (dec).

Example 2

*trans*-1a,2,3,4a,5,6-Hexahydro-7-methoxy-4H-naphth-[1,2-b]-1,4-oxazine hydrochloride

Employing the procedures substantially as described in Example 1, Steps A through D, but substituting for the starting material used therein an equimolar amount of 2-amino-5-methoxy-3,4-dihydronaphthalen-1(2H)-one hydrochloride, there were produced in sequence:

*Step A:* 2-chloroacetamido-5-methoxy-3,4-dihydronaphthalen-1(2H)-one, m.p. 147—149°C.

*Step B:* *trans*-2-chloroacetamido-5-methoxy-1,2,3,4-tetrahydronaphthalen-1-ol, m.p. 125—128°C.

*Step C:* *trans*-1a,2,4,4a,5,6-hexahydro-7-methoxynaphth[1,2-b]-1,4-oxazin-3-one, m.p. 294—295°C.

*Step D:* *trans*-1a,2,3,4a,5,6-hexahydro-7-methoxy-4H-naphth[1,2-b]-1,4-oxazine hydrochloride, m.p. 260°C.

Employing the procedure substantially as described in Example 1, Steps A through D but substituting

for the starting materials used in Step A thereof an equimolecular amount of each of the substituted 2-amino-3,4-dihydronaphthalen-1(2H)-ones described in Table I and the α-$R^5$-chloroacetyl chlorides also described in Table I there are produced the respective substituted *trans*-1a,2,3-4a,5,6-hexahydronaphth[1,2-b]-1,4-oxazines also described in Table I in accordance with the following reaction sequence:

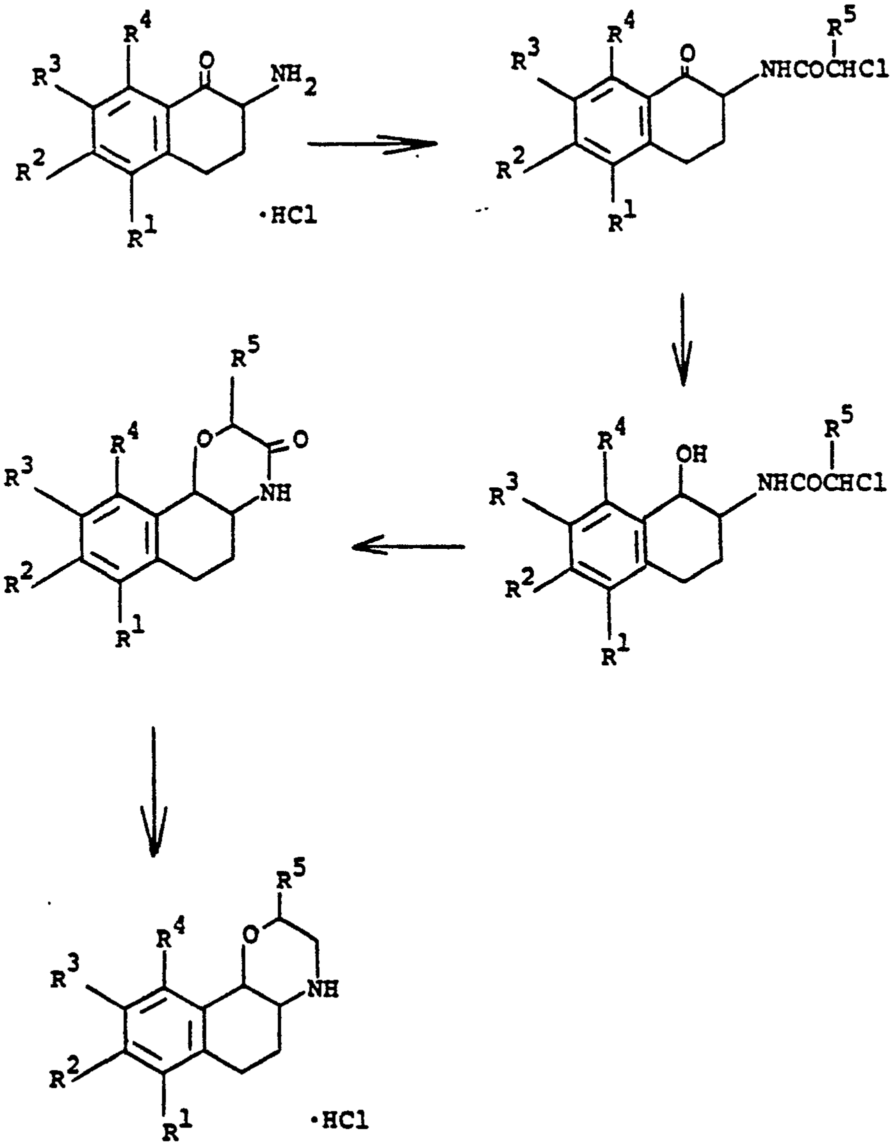

TABLE I

| $R^1$ | $R^2$ | $R^3$ | $R^4$ | $R^5$ |
|---|---|---|---|---|
| Cl | H | H | H | H |
| H | H | Cl | H | H |
| Cl | Cl | H | H | H |
| H | —Cl | H | H | H |
| $—CH_3$ | H | H | H | H |
| $—CH_3$ | $—CH_3$ | H | H | H |
| H | $—CH_3$ | H | H | H |
| H | H | $—CH_3$ | H | H |
| —F | H | H | H | H |
| —F | —F | H | H | H |
| H | —F | H | H | H |
| H | H | —F | H | H |
| H | $—OCH_3$ | H | H | H |
| H | H | $—OCH_3$ | H | $—CH_3$ |
| H | H | H | $—OCH_3$ | H |
| $—OCH_3$ | $—OCH_3$ | H | H | H |
| H | $—OCH_3$ | $—OCH_3$ | H | H |
| H | $—OC_2H_5$ | H | H | H |
| H | H | $—OC_3H_7$ | H | $—C_6H_5$ |
| $—OC_2H_5$ | H | H | H | $—C_3H_7—n$ |
| H | H | $—OCH_3$ | H | $—C_6H_5$ |

Example 3

*trans*-1a,2,3,4a,5,6-Hexahydro-4-benzyl-4H-naphth[1,2-b]-1,4-oxazine hydrochloride hemihydrate

*Step A:* Preparation of 2-benzamido-3,4-dihydronaphthalen-1(2H)-one

A solution of N-benzoyl homophenylalanine (4.4 g, 1.66 mole) in acetic anhydride (60 ml) was heated on the steam bath for 1/2 hr. The solvent was removed *in vacuo,* the resulting oil was dissolved in $CS_2$ and then added to a suspension of $AlCl_3$ (6.2 g, 4.6 mmoles) in $CS_2$ (60 ml). This mixture was heated at reflux for 1 hr, the solvent was removed *in vacuo* and ice was added to the residue. The reaction mixture was extracted with ethyl acetate, the ethyl acetate layer was washed with brine, dried over $Na_2SO_4$ and evaporated to dryness to afford 3.6 g (88%) of product, m.p. 175°C. After recrystallization from toluene it had m.p. 175—178°C.

*Step B:* Preparation of *trans*-2-Benzylamino-1,2,3,4-tetrahydronaphthalen-1-ol

A solution of 2-benzamido-3,4-dihydronaphthalen-1(2H)-one (3.0 g, 1.1 mmole) in THF (150 ml) was added dropwise to a suspension of $LiAlH_4$ (2.0 g) in THF (50 ml). The reaction was heated at reflux for 2 hr and then cooled to room temperature. Enough ethanol was added to destroy excess $LiAlH_4$ followed by 150 ml of Rochell's salt solution (20%). The resulting mixture was extracted with ethyl acetate, the ethyl acetate layer was washed with brine and dried over $Na_2SO_4$. Evaporation of the solvent afforded 2.5 g (88%) of the

product, m.p. 110—114°C. After recrystallization from cyclohexane it had m.p. 112—115°C.

*Step C:* Preparation of *trans*-1a,2,4,4a,5,6-Hexahydro-4-benzylnaphth[1,2-b]-1,4-oxazin-3-one

A bicarbonate solution (2.0 g in 75 ml of $H_2O$) was layered with an ethyl acetate solution (100 ml) containing trans-2-benzylamino-1,2,3,4-tetrahydronaphthalen-1-ol (2.0 g, 0.8 mmole), and the whole was stirred rapidly while chloroacetyl chloride (0.89 g, 0.8 mmole) was added dropwise. After 0.5 hr the ethyl acetate layer was separated, dried over $Na_2SO_4$ and evaporated. The resulting light purple solid was dissolved in DMF (10 ml) and added to a suspension of NaH (0.5 g of 53% mineral oil) in DMF (15 ml). After 1 hr the reaction mixture was poured into water (150 ml). The solid that separated was filtered and dried to yield 1.8 g (78%) of product, m.p. 185°C. After recrystallization from butyl chloride it had m.p. 185—187°C.

*Step D:* Preparation of *trans*-1a,2,3,4a,5,6-Hexahydro-4-benzyl-4H-naphth[1,2-b]-1,4-oxazine hydrochloride hemihydrate

This reaction was carried out substantially as described in Example 1, Step D using trans-1a,2,4,4a,5,6-hexahydro-4-benzylnaphth[1,2-b]-1,4-oxazin-3-one as the starting material. There was obtained a 64% yield of product, m.p. 262—265°C (dec.).

Example 4

*trans*-1a,2,3,4a,5,6-Hexahydro-4-allyl-4H-naphth[1,2-b]-1,4-oxazine hydrochloride

To a solution of *trans*-1a,2,3,4a,5,6-hexahydro-4H-naphth[1,2-b]-1,4-oxazine (1.0 g, 0.52 mmole) (Example 1, Step D) in DMF (15 ml) was added $K_2CO_3$ (1.1 g) and allyl bromide (0.95 g, 0.79 mmoles). The reaction mixture was stirred at room temperature for 6 hrs, and then concentrated *in vacuo* to a small volume. The reaction was diluted with water (40 ml) and extracted with ether (3 × 100 ml). The ether layer was washed with brine, dried over $Na_2SO_4$ and filtered. Ethanolic hydrochloric acid was added and the salt separated to give 0.77 g (55%) of product, m.p. 187—192°C.

Employing the procedure substantially as described in Example 4 but substituting for the naphthoxazine and allyl bromide used therein, equimolecular amounts of the naphthoxazines and alkyl halides (R—X) described in Table II, there were produced the N-alkyl-naphthoxazines, also described in Table II in accordance with the following reaction:

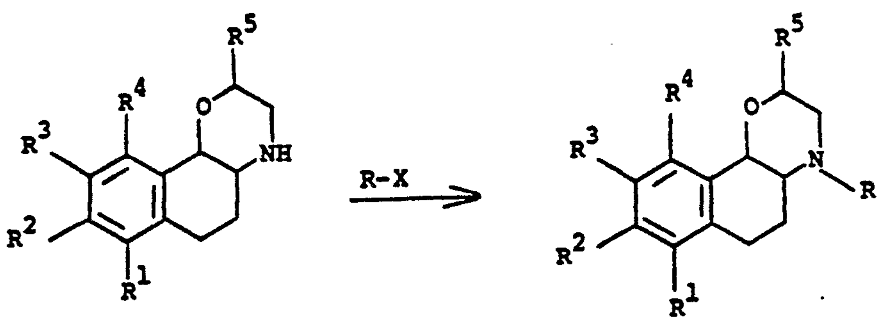


TABLE II

| X | R | $R^1$ | $R^2$ | $R^3$ | $R^4$ | HCl salt m.p. (°C) |
|---|---|---|---|---|---|---|
| Br | n-$C_3H_7$— | H | H | H | H | 259—261 |
| Br | $C_2H_5$— | —$OCH_3$ | H | H | H | 279—284 |
| Br | $CH_2$=$CHCH_2$— | —$OCH_3$ | H | H | H | 245—246 |

Similarly prepared are the compounds described in Table III.

TABLE III

| R | $R^1$ | $R^2$ | $R^3$ | $R^4$ | $R^5$ |
|---|---|---|---|---|---|
| $CH_3$— | H | H | H | H | H |
| $CH_3$— | —$OCH_3$ | H | H | H | H |
| $C_6H_5CH_2$— | H | H | H | H | H |
| $C_6H_5CH_2$— | —$OCH_3$ | H | H | H | H |
| $C_2H_5$— | —Cl | H | H | H | H |
| $C_2H_5$— | Cl | —Cl | H | H | H |
| $C_2H_5$— | H | —$CH_3$ | H | H | H |
| $C_2H_5$— | H | H | —$CH_3$ | H | —$CH_3$ |
| $C_2H_5$— | —F | —F | H | H | H |
| $C_3H_7$ | —$OCH_3$ | H | H | H | H |
| $C_3H_7$ | H | H | $OCH_3$ | H | H |
| $C_2H_5$— | H | —$OCH_3$ | H | H | H |
| $C_2H_5$— | H | H | H | —$OCH_3$ | H |
| $C_3H_7$ | —$OCH_3$ | —$OCH_3$ | H | H | H |
| $C_3H_7$ | H | —$OCH_3$ | —$OCH_3$ | H | H |
| i-$C_3H_7$— | H | H | H | —$OCH_3$ | H |
| $C_2H_5$— | H | H | —$OCH_3$ | H | —$C_6H_5$ |

Example 5

*trans*-1a,2,3,4a,5,6-Hexahydro-4-ethyl-9-methoxy-4H-naphth[1,2-b]-1,4-oxazine hydrochloride

*Step A:* Preparation of 7-methoxy-2-oximino-3,4-dihydronaphthalen-1(2H)-one

A mixture of potassium *tert*-butoxide (11.5 gm, 0.1 m), ether (400 ml), *tert*-butanol (15 ml) and absolute ethanol (15 ml) was refluxed for 1/2 hour to insure complete solution of the potassium *tert*-butoxide. To the hot solution was added 7-methoxy-1-tetralone (17.6 gms, 0.1 m) and the reaction mixture turned from yellow to dark brown. To this refluxing solution was added isopentyl nitrite (19 ml). During this addition external heating was not necessary because of the exothermic nature of the reaction. The reaction mixture was refluxed for 1/2 hour during which time the walls of the flask became coated with a brown solid. After cooling in an ice-bath, the solvent was removed by decantation. To the solid was added aqueous hydrogen chloride (1*N*, 200 ml). After stirring 1/2 hour, the brown solid was filtered to yield 10 gms (50%) of product.

*Step B:* Preparation of 2-Acetamido-7-methoxy-3,4-dihydronaphthalen-1(2H)-one

A mixture of 2-oximino-7-methoxy-3,4-dihydronaphthalen-1(2H)-one (8.7 gm, 0.04 m), palladium on carbon (10%, 1 gm) tetrahydrofuran (150 ml) and acetic anhydride (25 ml) was hydrogenated on a Parr apparatus for 3 hours. The catalyst was filtered, and the solvents were removed under reduced pressure to yield the product as an oil, which was used in the next step without further purification.

*Step C:* Preparation of *trans*-2-Acetamido-7-methoxy-1,2,3,4-tetrahydronaphthalen-1-ol

To a stirred solution of 2-acetamido-7-methoxy-3,4-dihydronaphthalen-1(2H)-one in absolute ethanol (100 ml) was added sodium borohydride (1.6 gms, 0.04 m) in portions. After stirring for 1/2 hour, acetic acid was added to decompose any excess sodium borohydride. The reaction mixture was poured onto water (150 ml) and this was extracted with ethyl acetate (3 × 150). The ethyl acetate was washed with aqueous saturated sodium chloride, and was dried over anhydrous sodium sulfate. After filtration, the ethyl acetate

was removed under reduced pressure (20 mm) to give a semisolid. The solid was slurried with ether and filtered to yield 3.3 gms (35%) of product as a white solid, m.p. 135°—140°C.

*Step D:* Preparation of *trans*-1a,2,4,4a,5,6-Hexahydro-4-ethyl-9-methoxynaphth[1,2-b]-1,4-oxazin-3-one

To a suspension of lithium aluminum hydride (2.8 gms, 0.07 m) in tetrahydrofuran (30 ml) at 0—5°C was added a slurry of *trans*-2-acetamido-7-methoxy-1,2,3,4-tetrahydronaphthalen-1-ol (2.16 gms, 0.009 m) in tetrahydrofuran (12 ml) and ethylene glycol dimethyl ether (8 ml). During the addition the internal temperature was kept below 10°C. After the addition was completed, the reaction mixture was refluxed for 1/2 hour. The reaction was cooled to 5—10°C, and the excess lithium aluminum hydride was hydrolyzed with isopropanol (4.8 ml) and saturated aqueous sodium sulfate (2.4 ml). After the addition of methylene chloride (50 ml), the inorganic salts were removed by filtration through filter cell. The solvent was removed under reduced pressure (20 mm) and the tan solid was dissolved in ethyl acetate (60 ml). To the stirred ethyl acetate solution was added water (90 ml) with sodium carbonate (9 gms) dissolved in it. To the biphasic mixture was added chloroacetyl chloride (1.5 ml) dropwise. The organic layer was separated and was washed with saturated aqueous sodium chloride. After drying over anhydrous sodium sulfate and filtration, the solvent was removed under reduced pressure (20 mm). The oil was dissolved in acetonitrile (7.5 m) and tetrahydrofuran (7.5 ml) and it was added to an ice cold (0—5°C) suspension of sodium hydride (0.75 gm, 50%) in tetrahydrofuran (20 ml). The excess sodium hydride was destroyed by addition of absolute ethanol, and the reaction mixture was poured onto water (100 ml). This was extracted with ethyl acetate, and the extract was washed with saturated aqueous sodium chloride. After drying over anhydrous sodium sulfate and filtration, the solvent was removed under reduced pressure (20 mm) to give 1.8 g (77%) of product.

*Step E:* Preparation of *trans*-1a,2,3,4a,5,6-Hexahydro-4-ethyl-9-methoxy-4H-naphth[1,2-b]-1,4-oxazine hydrochloride

To a slurry of lithium aluminum hydride (1.0 gms, 0.02 m) in tetrahydrofuran (20 ml) at 0—5°C was added *trans*-1a,2,4,4a,5,6-hexahydro-4-ethyl-9-methoxynaphth[1,2-b]-1,4-oxazin-3-one (1.8 gms, 0.007 m) in tetrahydrofuran (36 ml) and ethylene glycol dimethyl ether (24 ml). Upon completion of the addition, the reaction mixture was refluxed for 1/2 hour. After cooling to 0—5°C, the excess lithium aluminum hydride was hydrolyzed with isopropanol (4.8 ml) and saturated aqueous sodium sulfate (3 ml). After addition of methylene chloride (50 ml), the inorganic salts were collected by filtration through filter cell. The solvents were dried over anhydrous sodium sulfate. After filtration, the organic solvents were removed under reduced pressure (20 mm) to give an oil. The oil was dissolved in ether (50 ml) and ethanolic hydrogen chloride (2.0 ml, 7.2 *N*) was added. After decantation of the solvent, the solid was recrystallized from methanol to yield 500 mg of product, m.p. 273—275°C.

Employing the procedure substantially as described in Example 5, Steps A through E, but substituting for the starting material used therein, an equimolecular amount of α-tetralone, 6-methoxy-1-tetralone, 5,6-dimethoxy-1-tetralone, 8-benzyloxy-1-tetralone, 6-benzyloxy-1-tetralone, and 6,7-dimethoxy-1-tetralone, there were produced, respectively:

*trans*-1a,2,3,4a,5,6-hexahydro-4-ethyl-4H-naphth[1,2-b]-1,4-oxazine hydrochloride, m.p. 218—221°C (dec.); and

*trans*-1a,2,3,4a,5,6-hexahydro-4-ethyl-8-methoxy-4H-naphth[1,2-b]-1,4-oxazine hydrochloride, m.p. 210—213°C.

*trans*-1a,2,3,4a,5,6-hexahydro-4-ethyl-7,8-dimethoxy-4H-naphth[1,2-b]-1,4-oxazine hydrochloride, m.p. 184—185°C.

*trans*-1a,2,3,4a,5,6-hexahydro-10-benzyloxy-4-ethyl-4H-naphth[1,2-b]-1,4-oxazine, oil);

*trans*-1a,2,3,4a,5,6-hexahydro-8-benzyloxy-4-ethyl-4H-naphth[1,2-b]-1,4-oxazine, (oil); and

*trans*-1a,2,3,4a,5,6-hexahydro-8,9-dimethoxy-4-ethyl-4H-naphth[1,2-b]-1,4-oxazine hydrochloride, m.p. 287—289°C. (dec.).

Employing the procedure substantially as described in Example 5, Steps A through E but substituting for the acetic anhydride used in Step B thereof an approximately equimolar amount of propionic anhydride, there was produced in sequence: 2-propionamido-7-methoxy-3,4-dihydro-1(2H)-naphthalenone;

*trans*-2-propionamido-7-methoxy-1,2,3,4-tetrahydro-1-naphthalenol;

*trans*-1a,2,3,4a,5,6-hexahydro-9-methoxy-4-propylnaphth[1,2-b]-1,4-oxazine-3-one; and

*trans*-1a,2,3,4a,5,6-hexahydro-9-methoxy-4-propyl-4H-naphth[1,2-b]-1,4-oxazine hydrochloride, m.p. 237—241°C.

Similarly, from:

6-benzyloxy-7-methoxy-1-tetralone;

6-methoxy-7-benzyloxy-1-tetralone; and

6,7-dibenzyloxy-1-tetralone there are produced, respectively,

*trans*-1a,2,3,4a,5,6-hexahydro-8-methoxy-9-methoxy-4-propyl-4H-naphth[1,2-b]-1,4-oxazine hydrochloride

*trans*-1a,2,3,4a,5,6-hexahydro-8-methoxy-9-benzyloxy-4-propyl-4H-naphth[1,2-b]-1,4-oxazine hydrochloride; and

*trans*-1a,2,3,4a,5,6-hexahydro-8,9-dibenzyloxy-4-propyl-4H-naphth[1,2-b]-1,4-oxazine hydrochloride. Similarly, using benzoic anhydride there are prepared in sequence:
*trans*-2-benzamido-7-methoxy-1,2,3,4-tetrahydro-1-naphthalenol;
*trans*-1a,2,3,4a,5,6-hexahydro-9-methoxy-4-benzylnaphth[1,2-b]-1,4-oxazine-3-one; and
*trans*-1a,2,3,4a,5,6-hexahydro-9-methoxy-4-benzyl-4H-naphth(1,2-b]-1,4-oxazine hydrochloride.

Example 6

(+)- and (−)- *trans*-1a,2,3,4a,5,6-Hexahydro-4-ethyl-4H-naphth[1,2-b]-1,4-oxazine hydrochloride

*Step A:* Preparation of 2-Acetamido-3,4-dihydronaphthalen-1(2H)-one

A slurry of 2-oximino-3,4-dihydronaphthalen-1(2H)-one (8.75 gm, 0.05 m) acetic anhydride (25 ml), tetrahydrofuran (150 ml) and Pd/C (10%, 500 mg) was hydrogenated on a Parr apparatus for 3 hours. The catalyst was removed by filtration through diatomaceous earth. The organic solvents were evaporated under reduced pressure (20 mm) to yield the product as an oil, which was used in the next step without further purification.

Alternatively:

To a stirred biphasic mixture of aqueous sodium bicarbonate (10 gm in 100 ml) and methylene chloride (250 ml) was added 3,4-dihydro-2-aminonaphthalen-1(2H)-one hydrochloride (8.3 gms, 0.04 m). When solution was achieved, acetylchloride (3.9 gms, 0.05 m) was added dropwise. The reaction was stirred for 1 hour. The organic layer was separated, washed with saturated aqueous sodium chloride solution, and dried over anhydrous sodium sulfate. After filtration of the inorganics, the solvent was removed under reduced pressure (20 mm) to give the product as a dark semisolid which was used in the next step without purification.

*Step B:* Preparation of *trans*-2-Acetamido-1,2,3,4-tetrahydronaphthalen-1-ol

To a solution of the 2-acetamido-3,4-dihydronaphthalen-1(2H)-one from Step A in absolute ethanol (150 ml) was added sodium borohydride (2.0 gms, 0.05 m) in portions. The reaction mixture was stirred at room temperature for 1/2 hour. Acetic acid was added dropwise to destroy the excess sodium borohydride. The reaction mixture was poured into water and extracted with ethyl acetate (3 × 150 ml). The ethyl acetate was washed with saturated aqueous sodium chloride (150 ml) and dried over anhydrous sodium sulfate. After filtration the solvent was removed under reduced pressure to yield 5.5 gms (54%, over Steps A and B), of product as a solid.

*Step C:* Resolution of *trans*-2-Acetamido-1,2,3,4-tetrahydronaphthalen-1-ol *via* 1-α-methoxy mandelic acid esters

*Step 1:* Ester Formation and Separation

To a stirred mixture of *trans*-2-acetamido-1,2,3,4-tetrahydronaphthalen-1-ol (1.0 gm, 0.0049 m), N,N′-dicyclohexylcarbodiimide (1.7 gms, 0.0083 m) and 1-α-methoxymandelic acid (1.5 gm, 0.009 m) in methylene chloride (125 ml) was added 4-dimethylaminopyridine (0.2 gm). After filtration of the solid formed, the methylene chloride solution was flash chromatographed to give 450 mg of "top ester" and 400 mg of "bottom ester"; 52% and 45% yield respectively.

*Step 2:* Saponification to yield (+)-*trans*-2-acetamido-1,2,3,4-tetrahydronaphthalen-1-ol and (−)*trans*-2-acetamido-1,2,3,4-tetrahydronaphthalen-1-ol

To a solution of potassium hydroxide (0.52 gm, 0.009 m) in absolute ethanol (20 ml) was added the "top ester" (2.1 gm, 0.005 m) and water (10 drops). The mixture was heated in a hot water bath at 50°C until solution was achieved (10 mins). The reaction mixture was poured into water, and the solid (160 mg) was filtered. The aqueous layer was extracted with chloroform (3 × 150 ml). The chloroform was dried over anhydrous sodium sulfate. After filtration, the chloroform was removed under reduced pressure (20 mm) to give a purple solid. The solid was suspended in ether and filtered to yield 0.95 gms (78%) of product as a white solid with a rotation of $[\alpha]_D^{25} = +49.8°$ (C, 0.996 in ethanol).

To a solution of potassium hydroxide (0.39 gm, 0.007 m) in absolute ethanol (20 ml) was added the "bottom ester" (1.6 gm, 0.0045 m) and water (10 drops). The mixture was heated in a hot water bath at 50°C until solution was achieved (10 mins). The reaction mixture was poured into water and it was extracted with chloroform. The chloroform was dried over anhydrous sodium sulfate. After filtration, the chloroform was removed under reduced pressure (20 mm) to yield 0.7845 gms (85%) of product as a white solid; $[\alpha]_D^{25} = -41.6°$ (C, 0.0994 in ethanol).

*Step D:* Preparation of (+)- and (−)-*trans*-1a,2,4,4a,5,6-Hexahydro-4-ethylnaphth[1,2-b]-1,4-oxazin-3-one

To a suspension of lithium aluminum hydride (0.72 gm, 0.0189 m) in tetrahydrofuran (10 ml) at 0°C was added a suspension of (+)-*trans*-2-acetamido-1,2,3,4-tetrahydronaphthalen-1-ol (0.7 gms, 0.0034 m) in tetrahydrofuran (12 ml) and ethylene glycol dimethyl ether (8 ml). During the addition the internal temperature was kept below 10°C. After the addition was completed, the reaction mixture was heated to reflux for 1/2 hour. After cooling the reaction to 5—10°C, the excess lithium aluminum hydride was

hydrolyzed with isopropanol (1.6 ml) and saturated aqueous sodium sulfate (0.8 ml). After addition of methylene chloride (50 ml), the inorganic salts were removed under reduced pressure, and the oil was dissolved in ethyl acetate (20 ml). To the stirred ethyl acetate solution was added water (20 ml) with sodium carbonate (3.04 g) dissolved in it. To the biphasic mixture was added chloroacetyl chloride (0.48 ml). The organic layer was separated and washed with saturated aqueous sodium sulfate. After filtration, the ethyl acetate was removed under reduced pressure (20 mm) to give an oil. The oil was dissolved in acetonitrile (2.5 ml) and tetrahydrofuran (2.5 ml). This solution was added to a suspension of sodium hydride (0.24 gm, 50%) in tetrahydrofuran (10 ml) at 0—5°C. After the addition was completed, the excess sodium hydride was destroyed by adding absolute ethanol. The reaction mixture was poured into water and was extracted with ethyl acetate. After the ethyl acetate was washed with saturated aqueous sodium chloride, it was dried over anhydrous sodium sulfate. After filtration, the ethyl acetate was removed under reduced pressure (20 mm) to yield an oil which crystallized to give 550 mg (70%) of (+)-product, m.p. 104°—106°C.

The (−) isomer was produced in the same manner using the same reaction conditions to give 450 mg (56%) of (−)-product, m.p. 97°—100°C.

*Step E:* Preparation of (+)- and (−)-*trans*-1a,2,3,4a,5,6-Hexahydro-4-ethyl-4H-naphth[1,2-b]-1,4-oxazine hydrochloride

To a suspension of lithium aluminum hydride (0.5 gm, 0.01 m) in tetrahydrofuran (10 ml) at 0—5°C was added (+)-*trans*-1a,2,4,4a,5,6-hexahydro-4-ethyl-naphth[1',2-b]-1,4-oxazin-3-one (0.55 gm, 0.0024 m) in tetrahydrofuran (12 ml) and ethylene glycol dimethylether (8 ml). Upon completion of the addition, the reaction mixture was refluxed for 1/2 hour. The reaction mixture was cooled to 0—5°C and the excess lithium aluminum hydride was hydrolyzed with isopropanol (1.6 ml) and saturated aqueous sodium sulfate (0.8 ml). After addition of methylene chloride (50 ml), the inorganic salts were removed by filtration through filter cell. The solution was dried by anhydrous sodium sulfate. After filtration, the organic solvents were removed under reduced pressure (20 min) to give an oil. The oil was dissolved in ether (100 ml) and ethanolic hydrogen chloride (1.5 ml, 7.2 *N*) was added. The precipitated solid was recrystallized from methanol to give 149 mg (25%) of (+)-product, m.p. 233—237°C, $[\alpha]_D^{25}$ = +56.56° (C, 0.944 in ethanol).

(−)-*trans*-1a,2,3,4a,5,6-Hexahydro-4-ethyl-4H-naphth-[1,2-b]-1,4-oxazine hydrochloride was produced in a similar manner using lithium aluminum hydride (0.35 gm, 0.009 m) and (−)-*trans*-1a,2-4,4a,5,6-hexahydro-4-ethylnaphth[1,2-b]-1,4-oxazin-3-one (0.45 gm, 0.002 m) to give 130 mg (26%) of (−)-product; m.p. 230—234°C, $[\alpha]_D^{25}$ = −57.10° (C, 0.904 in ethanol).

Example 7

(+)-*trans*-1a,2,3,4a,5,6-Hexahydro-9-methoxy-4-propyl-4H-naphth[1,2-b]-1,4-oxazine hydrochloride

*Step A:* Esterification of *trans*-2-propionamido-7-methoxy-1,2,3,4-tetrahydro-1-naphthalenol with 1-α-methoxymandelic acid and separation of the diastereomeric esters.

A mixture of equal molar quantities of trans-2-propionamido-7-methoxy-1,2,3,4-tetrahydro-1-naphthalenol, N,N'-dicyclohexylcarbodiimide, and α-methoxymandelic acid in methylene chloride, containing a catalytic amount of 4-dimethylaminopyridine was stirred at room temperature for 1 hour. The reaction mixture was filtered and subjected to medium pressure chromatography over silica gel. By collecting the proper fractions there were obtained the pure (+) and (−) enantiomeric esters.

*Step B:* Preparation of (+)-*trans*-2-propionamido-7-methoxy-1,2,3,4-tetrahydro-1-naphthalenol

Material obtained from one of the pure fractions obtained in Step A was dissolved in a solution of potassium hydroxide (slight molar excess) in ethanol and heated at reflux for 10 minutes. The cooled reaction mixture was poured into water, neutralized with dilute hydrochloric acid and the resulting solid was recovered by filtration. Recrystallization from ethyl acetate afforded the subject compound, m.p. 162—163°C $[\alpha]_D$ + 71.02 (C 0.105 EtOH).

Anal. Calc. for $C_{14}H_{19}NO_3$: C, 67.44; H, 7.68; N, 5.61.

Found: C, 67.73; H, 7.93; N, 5.55.

*Step C:* Preparation of (−)-trans-1a,2,4,4a,5,6-Hexahydro-9-methoxy-4-propylnaphth[1,2-b]-1,4-oxazin-3-one

Using essentially the procedure described in Example 6, Step D but employing the product from Step B above as starting material the subject compound was obtained as a solid, 94—96°C, $[\alpha]_D$ −36.94 (C, 0.0896, EtOH).

Anal. calcd. for $C_{16}H_{21}NO_3$: C, 69.79; H, 7.69; N, 5.09.

Found: C, 69.88; H, 8.02, N, 4.96.

*Step D:* Preparation of (+)-trans-1a,2,3,4a,5,6-Hexahydro-9-methoxy-4-propyl-4H-naphth[1,2-b]-1,4-oxazine hydrochloride

Using essentially the same procedure as described in Example 6, Step E but employing the product from Step C above as starting material, the subject compound was obtained as a solid m.p. 231—233°C, $[\alpha]_D$ +47.28 (C 0.105, EtOH).

Anal. Calcd. for $C_{16}H_{23}NO_2$·HCl: C, 64.52; H, 8.12; N, 4.70.
Found: C, 64.24; H, 8.23; N, 4.64.

Employing the procedure substantially as described in Example 7, Steps B, C and D but employing as starting material the other diastereomeric ester obtained in Step A of Example 7, there was obtained in sequence:
(−)-*trans*-2-propionamido-7-methoxy-1,2,3,4-tetrahydro-1-naphthalenol;
(+)-*trans*-1a,2,4,4a,5,6-hexahydro-9-methoxy-4-propylnaphth[1,2-b]-1,4-oxazin-3-one; and
(−)-*trans*-1a,2,3,4a,5,6-hexahydro-9-methoxy-4-propyl-4H-naphth[1,2-b]-1,4-oxazine hydrochloride, m.p. 231—233°C, $[\alpha]_D$ −47.44 (0.0978, EtOH).
Anal. Calcd. for $C_{16}H_{23}NO_2$.HCl: C, 64.52; H, 8.12; N, 4.70.
Found: C, 64.68; H, 8.37; N, 4.66.

## Example 8

(+)-*trans*-1a,2,3,4a,5,6-Hexahydro-9-methoxy-4-ethyl-4H-naphth[1,2-b]-1,4-oxazine hydrochloride

Employing the procedures substantially as described in Example 7, Step A, but substituting for the propionamido compound used therein, an equimolar amount of the acetamido homolog, there were obtained the separated, pure diastereomeric esters.

Taking one of the foregoing esters through the procedures as described in Example 7, Steps B, C and D there were produced in sequence:
(+)-*trans*-2-acetamido-7-methoxy-1,2,3,4-tetrahydro-1-naphthalenol;
*trans*-1a,2,4,4a,5,6-hexahydro-9-methoxy-4-ethylnaphth[1,2-b]-1,4-oxazin-3-one; and
(+)-*trans*-1a,2,3,4a,5,6-hexahydro-9-methoxy-4-ethyl-4H-naphth[1,2-b]-1,4-oxazine hydrochloride, m.p. 286—288°C (dec.).
Anal. Calcd. for $C_{15}H_{21}NO_2HCl$: C, 63.48; H, 7.82; N, 4.94.
Found: C, 63.08; H, 8.05; N, 5.01.

From the other diastereomeric ester of Example 8, using the procedures described in Example 7, Steps B, C and D, there were obtained in sequence: (−)-trans-2-acetamido-7-methoxy-1,2,3,4-tetrahydro-1-naphthalenol;
(−)-*trans*-1a,2,4,4a,5,6-hexahydro-9-methoxy-4-ethylnaphth[1,2-b]-1,4-oxazin-3-one; and
(−)-trans-1a,2,3,4a,5,6-hexahydro-9-methoxy-4-ethyl-4H-naphtha[1,2-b]-1,4-oxazine hydrochloride, 282—283°C (dec.).
Anal. Calcd. for $C_{15}H_{21}NO_2$.HCl: C, 63.48; H, 7.82; N, 4.94.
Found: C, 63.22; H, 8.06; N, 4.91.

Employing the procedures used in Example 6, 7 and 8, the other novel *trans* compounds of this invention are produced as optically pure products, such as (+) and (−)-*trans*-1a,2,3,4a,5,6-hexahydro-9-methoxy-4-benzyl-4H-naphth[1,2-b]-1,4-oxazine hydrochloride.

## Example 9

*trans*-1a,2,3,4a,5,6-Hexahydro-7-hydroxy-4-ethyl-4H-naphth[1,2-b]-1,4-oxazine hydrochloride

An intimate mixture of pyridine hydrochloride (312 mg, 0.27 mmole) and *trans*-1a,2,3,4a,5,6-hexahydro-4-ethyl-7-methoxy-4H-naphth[1,2-b]-1,4-oxazine hydrochloride (250 mg, 0.09 mmole) was heated at 200°C for 5 hours. The cooled reaction mixture was made slightly basic with $NH_4OH$ and then extracted with chloroform (3 × 50 ml). The organic phase was washed with brine, dried over $MgSO_4$, and then concentrated to dryness *in vacuo.* The resulting solid was dissolved in ethyl acetate and some 4*N* ethanolic hydrochloric acid was added which caused the precipitation of the product. The yield was 60 mg (25%), m.p. 294—297°C (dec.) (isopropanol).

Similarly, but omitting the treatment with ethanolic hydrogen chloride, there were prepared from the corresponding methoxy compounds:
*trans*-1a,2,3,4a,5,6-hexahydro-4-ethyl-9-hydroxy-4H-naphth[1,2-b]-1,4-oxazine, m.p. 223—225°C;
*trans*-1a,2,3,4a,5,6-hexahydro-4-propyl-9-hydroxy-4H-naphth[1,2-b]-1,4-oxazine, m.p. 164—166°C;
(+)-*trans*-1a,2,3,4a,5,6-hexahydro-4-ethyl-9-hydroxy-4H-naphth[1,2-b]-1,4-oxazine, m.p. 165—168°C, $[\alpha]_D$ + 51.77, (C, 0.101, EtOH);
(−)-*trans*-1a,2,3,4a,5,6-hexahydro-4-ethyl-9-hydroxy-4H-naphth[1,2-b]-1,4-oxazine, m.p. 164—166°C, $[\alpha]_D$ −45.45, (C, 0.101, EtOH);
(+)-*trans*-1a,2,3,4a,5,6-hexahydro-9-hydroxy-4-propyl-4H-naphth[1,2-b]-1,4-oxazine, m.p. 158—160°C, $[\alpha]_D$ +59.54, (C, 0.0964, EtOH); and
(−)-*trans*-1a,2,3,4a,5,6-hexahydro-9-hydroxy-4-propyl-4H-naphth[1,2-b]-1,4-oxazine, m.p. 158—161°C, $[\alpha]_D$ −62.63, (C, 0.0942, EtOH).

Employing the procedure substantially as described in Example 9, but substituting for the *trans*-1a,2,3,4a,5,6-hexahydro-4-ethyl-7-methoxy-4H-naphth[1,2-b]-1,4-oxazine hydrochloride used as starting material therein, an equimolar amount of the alkoxynaphthoxazines described in Table IV, there are produced the corresponding hydroxynaphthoxazines, also described in Table IV in accordance with the following reaction:

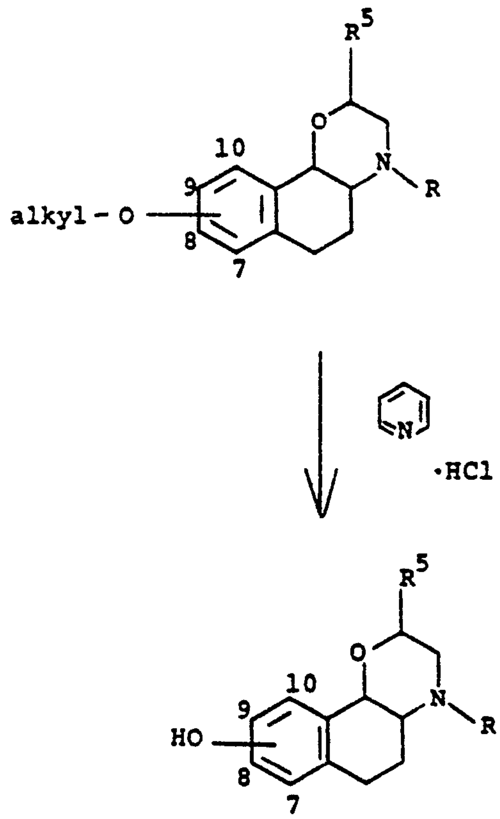
·HCl

TABLE IV

| STARTING MATERIALS | | | | | PRODUCTS | | | | |
|---|---|---|---|---|---|---|---|---|---|
| R | 7 | 8 | 9 | 10 | 7 | 8 | 9 | 10 | $R^5$ |
| $CH_2{=}CHCH_2$— | —$OCH_3$ | H | H | H | —OH | H | H | H | H |
| $CH_3$— | —$OCH_3$ | H | H | H | —OH | H | H | H | H |
| $C_6H_5CH_2$— | —$OCH_3$ | H | H | H | —OH | H | H | H | H |
| $C_3H_7$— | —$OCH_3$ | H | H | H | —OH | H | H | H | H |
| $C_3H_7$— | H | H | —$OCH_3$ | H | H | H | —OH | H | —$CH_3$ |
| $C_2H_5$— | H | H | —$OCH_3$ | H | H | H | —OH | H | —$C_6H_5$ |
| i-$C_3H_7$— | H | H | H | —$OCH_3$ | H | H | H | —OH | H |
| H | H | H | —$OCH_3$ | H | H | H | —OH | H | H |
| H | H | H | H | —$OCH_3$ | H | H | H | —OH | H |
| H | —$OCH_3$ | H | H | H | —OH | H | H | H | H |
| H | H | H | —$OC_3H_7$ | H | H | H | —OH | H | H |
| H | —$OC_2H_5$ | H | H | H | —OH | H | H | H | H |

Example 10

*trans*-1a,2,3,4a,5,6-Hexahydro-10-hydroxy-4-ethyl-4H-naphth[1,2-b]-1,4-oxázine hydrochloride

The 10-benzyloxy material from Example 5 was dissolved in $C_2H_5OH$:THF (1:1 v/v), 10% palladium on carbon catalyst was added and the reaction mixture was hydrogenated on a Parr apparatus at 25 psi until hydrogen uptake ceased. The reaction mixture was removed from the Parr, filtered, and the solvent was removed *in vacuo.* The residue was purified by medium pressure chromatography using $CHCl_3.CH_3OH$ (9:1 v/v) to elute. The subject compound was obtained as a white solid (hydrogen chloride gas added to an ethanol solution), m.p. 262—265°C (dec.).

Employing the procedure substantially as described in Example 10, but starting with the 8-benzyloxy compound, also from Example 5, there was produced *trans*-1a,2,3,4a,5,6-hexahydro-8-hydroxy-4-ethyl-4H-naphth[1,2-b]-1,4-oxazine, m.p. 187—191°C.

Employing the procedure substantially as described in Example 10 but starting with

*trans*-1a,2,3,4a,5,6-hexahydro-8-benzyloxy-9-methoxy-4-propyl-4H-naphth[1,2-b]-1,4-oxazine hydrochloride;

*trans*-1a,2,3,4a,5,6-hexahydro-8-methoxy-9-benzyloxy-4-propyl-4H-naphth[1,2-b]-1,4-oxazine hydrochloride; and

*trans*-1a,2,3,4a,5,6-hexahydro-8,9-dibenzyloxy-4-propyl-4H-naphth[1,2-b]-1,4-oxazine hydrochloride; there are produced respectively:

*trans*-1a,2,3,4a,5,6-hexahydro-8-hydroxy-9-methoxy-4-propyl-4H-naphth[1,2-b]-1,4-oxazine hydrochloride;

*trans*-1a,2,3,4a,5,6-hexahydro-8-methoxy-9-hydroxy-4-propyl-4H-naphth[1,2-b]-1,4-oxazine hydrochloride; and

*trans*-1a,2,3,4a,5,6-hexahydro-8,9-dihydroxy-4-propyl-4H-naphth[1,2-b]-1,4-oxazine hydrochloride.

Employing the procedure substantially as described in Example 10 but starting with the (+) or (−)-*trans*-1a,2,3,4a,5,6-hexahydro-9-methoxy-4-benzyl-4H-naphth[1,2-b]-1,4-oxazine from Example 8 there are produced.

(+)-*trans*-1a,2,3,4a,5,6-hexahydro-9-methoxy-4H-naphth[1,2-b]-1,4-oxazine hydrochloride; and

(−)-*trans*-1a,2,3,4a,5,6-hexahydro-9-methoxy-4H-naphth[1,2-b]-1,4-oxazine hydrochloride, respectively.

Example 11

*trans*-1a,2,3,4a,5,6-hexahydro-9-acetoxy-4-propyl-4H-naphth[1,2-b]-1,4-oxazine hydrochloride.

A mixture of *trans*-1a,2,3,4a,5,6-hexahydro-9-hydroxy-4-propyl-4H-naphth[1,2-b]-1,4-oxazine (0.7 g., 2.8 mmole), acetic anhydride (6 ml) and 4-dimethylaminopyridine (10 mg) was stirred and heated at 85—90°C for 1 hour. Most of the solvent was removed *in vacuo* and the residue was taken up in ethyl acetate (25 ml). Addition of ethanolic hydrogen chloride afforded the subject compound in 54% yield, m.p. 218—220° ($CH_3CN$).

Anal. calcd. for $C_{17}H_{23}NO_3$.HCl: C, 62.66; H, 7.42; N, 4.30.

Found: C 62.25; H, 7.56; N, 4.40.

Example 12

*trans*-1a,2,3,4a,5,6-Hexahydro-9-pivaloyloxy-4-propyl-4H-naphth[1,2-b]-1,4-oxazine hydrochloride.

This compound was prepared by essentially the same procedure described in Example 11 by substituting trimethylacetic anhydride for the acetic anhydride used in that example. The yield was 80%; m.p. 248—250°.

Anal. Calcd. for $C_{20}H_{29}NO_3$.HCl: C, 65.29; H, 8.22; N, 3.81.

Found: C, 65.18; H, 8.57; N, 3.66.

Example 13

Preparation of *trans*-1a,2,3,4a,5,6-Hexahydro-9-dimethylcarbamoyloxy-4-propyl-4H-naphth[1,2-b]-1,4-oxazine maleate

To a cooled (10°), stirred, suspension of NaH (0.2 g., 0.004 m) in THF (10 ml) was added a solution of *trans*-1a,2,3,4a,5,6-hexahydro-9-hydroxy-4-propyl-4H-naphth[1,2-b]-1,4-oxazine (1.0 g., 0.004 m) in THF (25 ml) over a period of 15 minutes. The reaction mixture was stirred one hour at 10—15° and then dimethylcarbamyl chloride (0.43 g., 0.004 m) was added. The reaction was stirred at room temperature for 18 hours, 250 ml of water were added and the solution was extracted with ethyl acetate (2 × 75 ml). The ethyl acetate extracts were dried over $Na_2SO_4$ and then concentrated *in vacuo.* The resulting oil was dissolved in ether and treated with an equivalent quantity of maleic acid in isopropanol. The resulting solid was recrystallized from ethyl acetate to afford the subject compound, melting point 140—142°C.

Employing the procedures substantially as described in Examples 11, 12 and 13 but substituting for the acid anhydrides, carbamyl chloride and the hydroxy-naphthoxazines used therein, comparable amounts of the anhydride of formula $(R^7CO)_2O$ or carbamyl chloride of formula $R^7COCl$ and the hydroxy-naphthoxazines identified in Table V, there are produced the acyloxynaphthoxazines also described in Table V, in accordance with the following reaction scheme:

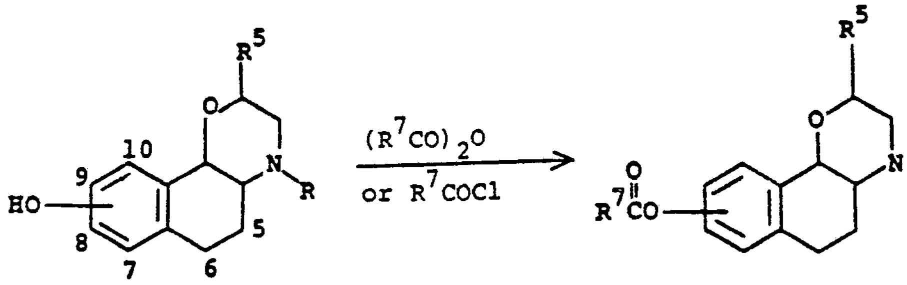

$(R^7CO)_2O$
or $R^7COCl$

TABLE V

| | | STARTING COMPOUND | | | | FINAL PRODUCT | | | | |
|---|---|---|---|---|---|---|---|---|---|---|
| Isomer | R | 7 | 8 | 9 | 10 | 7 | 8 | 9 | 10 | $R^5$ |
| (±)-trans | $-C_2H_5$ | H | H | —OH | H | H | H | $-O\overset{\overset{O}{\|}}{C}CH_3$ | H | H |
| (+)-trans | $-C_2H_5$ | H | H | —OH | H | H | H | $-O\overset{\overset{O}{\|}}{C}CH_3$ | H | H |
| (−)-trans | $-C_{25}$ | H | H | —OH | H | H | H | $-O\overset{\overset{O}{\|}}{C}CH_3$ | H | H |
| (+)-trans | $-C_3H_7$ | H | H | —OH | H | H | H | $-O\overset{\overset{O}{\|}}{C}H-CH_3$ | H | H |
| (−)-trans | $-C_3H_7$ | H | H | —OH | H | H | H | $-O\overset{\overset{O}{\|}}{C}-CH_3$ | H | H |
| (±)-trans | $-C_2H_5$ | H | H | —OH | H | H | H | $-O\overset{\overset{O}{\|}}{C}C(CH_3)_3$ | H | H |
| (+)-trans | $-C_2H_5$ | H | H | —OH | H | H | H | $-O\overset{\overset{O}{\|}}{C}C(CH_3)_3$ | H | H |
| (−)-trans | $-C_2H_5$ | H | H | —OH | H | H | H | $-O\overset{\overset{O}{\|}}{C}C(CH_3)_3$ | H | H |
| (+)-trans | $-C_3H_7$ | H | H | —OH | H | H | H | $-O\overset{\overset{O}{\|}}{C}C(CH_3)_3$ | H | H |
| (−)-trans | $-C_3H_7$ | H | H | —OH | H | H | H | $-O\overset{\overset{O}{\|}}{C}C(CH_3)_3$ | H | H |

TABLE V (continued)

| Isomer | R | Starting compound 7 | Starting compound 8 | Starting compound 9 | Starting compound 10 | Final product 7 | Final product 8 | Final product 9 | Final product 10 | Final product $R^5$ |
|---|---|---|---|---|---|---|---|---|---|---|
| (±)-trans | $—C_2H_5$ | H | H | —OH | H | H | H | $—O\overset{O}{\overset{\|}{C}}CH_2C_6H_5$ | H | H |
| (+)-trans | $—C_2H_5$ | H | H | —OH | H | H | H | $—O\overset{O}{\overset{\|}{C}}CH_2C_6H_5$ | H | H |
| (−)-trans | $—C_2H_5$ | H | H | —OH | H | H | H | $—O\overset{O}{\overset{\|}{C}}CH_2C_6H_5$ | H | H |
| (±)-trans | $—C_3H_7$ | H | H | —OH | H | H | H | $—O\overset{O}{\overset{\|}{C}}CH_2C_6H_5$ | H | H |
| (+)-trans | $—C_3H_7$ | H | H | —OH | H | H | H | $—O\overset{O}{\overset{\|}{C}}CH_2C_6H_5$ | H | H |
| (−)-trans | $—C_3H_7$ | H | H | —OH | H | H | H | $—O\overset{O}{\overset{\|}{C}}CH_2C_6H_5$ | H | H |
| (±)-trans | $—C_2H_5$ | —OH | H | H | H | $—O\overset{O}{\overset{\|}{C}}CH_3$ | H | H | H | H |
| (±)-trans | $—C_2H_5$ | H | H | H | —OH | H | H | H | $—O\overset{O}{\overset{\|}{C}}CH_2C_6H_5H$ | |
| (±)-trans | $—C_2H_5$ | H | —OH | H | H | H | $—O\overset{O}{\overset{\|}{C}}C_6H_5$ | H | H | H |

TABLE V (continued)

| | STARTING COMPOUND | | | | | FINAL PRODUCT | | | | |
|---|---|---|---|---|---|---|---|---|---|---|
| Isomer | R | 7 | 8 | 9 | 10 | 7 | 8 | 9 | 10 | $R^5$ |
| (±)-trans | $-CH_2CH{=}CH_2$ | —OH | H | H | H | $-O\overset{\overset{O}{\|}}{C}C_6H_{11}$ | H | H | H | H |
| (±)-trans | $-CH_3$ | —OH | H | H | H | 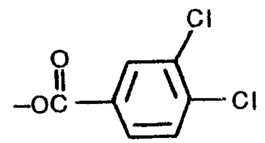 | H | H | H | H |
| (±)-trans | $-CH_2C_6H_5$ | —OH | H | H | H | 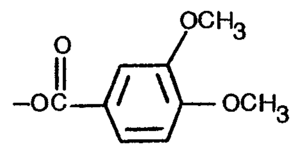 | H | H | H | H |
| (±)-trans | $-C_3H_7$ | —OH | H | H | H | 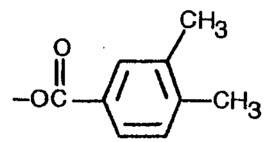 | H | H | H | H |
| (±)-trans | $-C_3H_7-i$ | H | H | H | —OH | H | H | H | $-O\overset{\overset{O}{\|}}{C}CH_2$ | H |
| (±)-trans | H | H | H | —OH | H | H | H | | 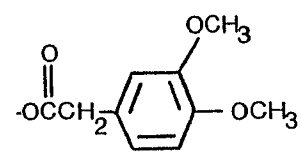 | H |
| (±)-trans | H | H | H | H | H | H | H | | 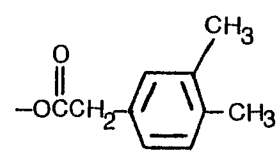 | H |

TABLE V (continued)

| | | STARTING COMPOUND | | | | FINAL PRODUCT | | | | |
|---|---|---|---|---|---|---|---|---|---|---|
| Isomer | R | 7 | 8 | 9 | 10 | 7 | 8 | 9 | 10 | $R^5$ |
| (±)-trans | $-C_3H_7$ | H | H | —OH | H | H | H | $-O\overset{O}{\overset{\|}{C}}CH_3$ | H | $-CH_3$ |
| (±)-trans | $-C_2H_5$ | H | H | —OH | H | H | H | $-O\overset{O}{\overset{\|}{C}}CH_3$ | H | $-C_6H_5$ |
| (±)-trans | $-C_2H_5$ | H | H | —OH | H | H | H | $-O\overset{O}{\overset{\|}{C}}N(CH_3)_2$ | H | H |
| (±)-trans | $-C_2H_7$ | H | H | —OH | H | H | H | 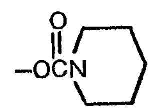 | H | H |
| (±)-trans | $-C_2H_7$ | H | H | —OH | H | H | H | 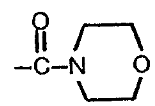 | H | H |
| (±)-trans | $-C_2H_7$ | H | H | —OH | H | H | H | 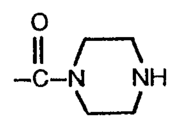 | H | H |
| (±)-trans | $-C_2H_7$ | H | H | —OH | H | H | H | 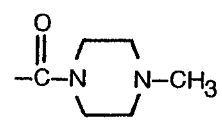 | H | H |
| (±)-trans | $-C_2H_7$ | H | H | —OH | H | H | H | $-\overset{O}{\overset{\|}{C}}-NHCH_3$ | H | H |

EXAMPLE 14

Pharmaceutical Composition

A typical tablet containing 100 mg of active ingredient per tablet is prepared by mixing together with the active ingredient, calcium phosphate, lactose and starch in the amounts shown in the table below. After these ingredients are thoroughly mixed, the appropriate amount of magnesium stearate is added and the dry mixture blended for an additional three minutes. This mixture is then compressed into tablets.

Tablet Formula

| Ingredient | Mg. per Tablet |
|---|---|
| *Trans*-1a,2,3,4a,5,6-hexahydro-9-hydroxy-4-propyl-4H-naphth-[1,2-b]-1,4-oxazine hydrochloride | 100 mg |
| Calcium phosphate | 52 mg |
| Lactose | 60 mg |
| Starch | 10 mg |
| Magnesium Stearate | 1 mg |

Similarly prepared are tablets comprising as active ingredient any of the other novel compounds described herein.

Important compounds of the present invention are *trans*-1a,2,3,4a,5,6-hexahydro-9-methoxy-4-propyl-4H-naphth[1,2-b]-1,4-oxazine, and the corresponding hydroxy compound, as racemates, or as the (±)-enantiomers with structural formula:

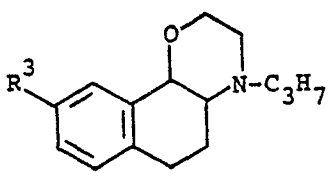

or a pharmaceutically acceptable salt thereof, wherein $R^3$ is —OH or —$OCH_3$.

These compounds are prepared by the following additional processes:

a) Reduction of a 4-propionyl group

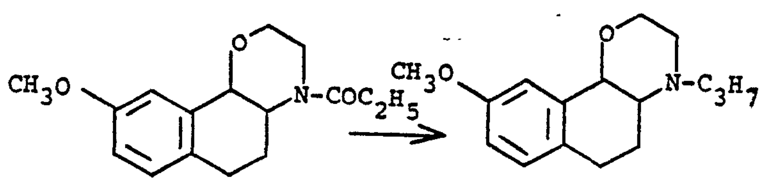

The reduction is accomplished with a complex metal hydride such as lithium aluminum hydride in an ethereal solvent such as THF, diethyl ether, or dimethoxyethane, preferably THF at about 25°C to reflux temperature for about ½ to 4 hours.

b) Reductive Alkylation of a 4-hydrogen compound

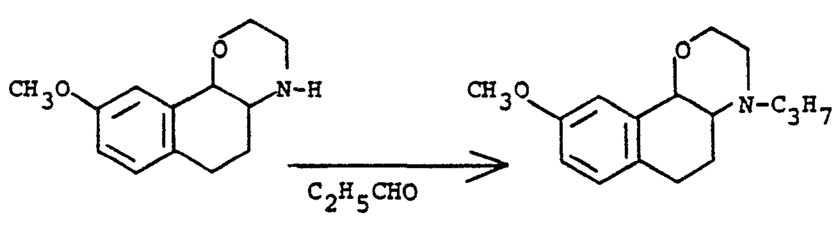


The reductive alkylation is accomplished by treating the compound, unsubstituted at nitrogen, with propionaldehyde, a noble metal hydrogenation catalyst such as palladium on carbon, palladium on barium

sulfate, platinum oxide or the like and hydrogen in an inert solvent such as $C_{1-3}$ alkanol, preferably ethanol, for about 2 to 10 hours at about 15°C to reflux temperature, preferably at room temperature.

Alternatively, the reductive alkylation is performed with propionaldehyde, and sodium cyanoborohydride in an inert solvent such as $C_{1-3}$ alkanol, preferably methanol for about 0.5 to 3 hours at about 15°C to reflux temperature, preferably about room temperature.

c) Double Condensation Tetrahydro-oxazine Ring Formation

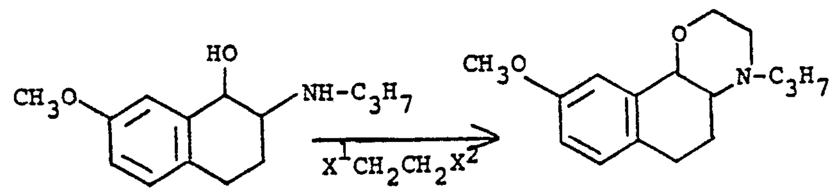


where $X^1$ and $X^2$ are the same or different and represent halo, such as bromo or iodo, benzenoid aromatic sulfonyloxy, such as toluenesulfonyloxy, or benzenesulfonyloxy, $C_{1-3}$ alkanesulfonyloxy, such as methanesulfonyloxy, ethanesulfonyloxy, or propanesulfonyloxy.

The double condensation is performed by treating the propylamino-alcohol with a 1,2-disubstituted ethane wherein the substituents are good leaving groups such as described, with an alkali metal hydroxide such as sodium or potassium hydroxide in an inert organic solvent such as acetonitrile, methylene chloride, chloroform or the like, preferably in the presence of a phase transfer agent such as methyl tricaprylyl ammonium chloride at about 15°C to reflux temperature for about 18 to 36 hours.

d) Closure of the Tetrahydro-oxazine ring.

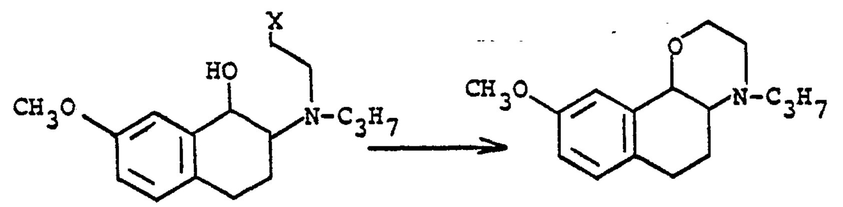

wherein X is a facile leaving group such as bromo, iodo, benzenoid arylsulfonyloxy such as toluenesulfonyloxy, benzenesulfonyloxy, or $C_{1-3}$ alkanesulfonyloxy such as methanesulfonyloxy, ethanesulfonyloxy or propanesulfonyloxy.

The ring closure is peformed by treatment of the starting material with a strong base, such as sodium hydride, potassium t-butoxide or the like in an inert organic solvent such as THF, or acetonitrile or mixtures thereof with a trace of a $C_{1-3}$ alkanol such as ethanol. The reaction proceeds at room temperature but any temperature from about 15°C to reflux may be employed for periods of about 0.5 hr to 6 hours.

An alternate procedure comprises the ring closure of compound *7* wherein X is —OH and is accomplished by treatment of *7* (X=OH) with diethylazodicarboxylate and triphenylphosphine in an anhydrous aprotic solvent such as ether, or THF at room temperature or below, to about −10°C, for about 0.5 to 12 hours.

e) Reduction of a 4-allyl group

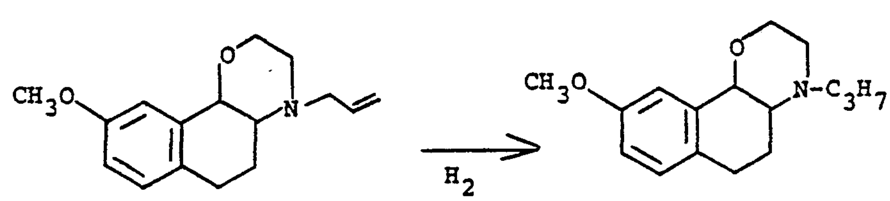


This reduction comprises treating the alkyl compound with hydrogen in the presence of a noble metal catalyst such as platinum oxide, palladium on carbon, palladium on barium sulfate or the like in a $C_{1-3}$ alkanol, preferably ethanol at about 15°C to reflux temperature until one mole of hydrogen/mole of starting material is absorbed.

f) Methylation of the Phenolic Hydroxyl

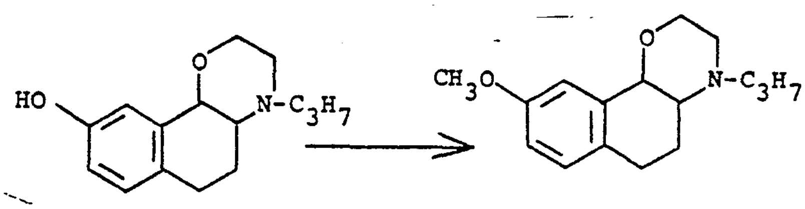

The methylation is performed with methyliodide in the presence of a strong base such as sodium hydride or potassium t-butoxide in an ethereal solvent such as THF, ether, dimethoxyethane or the like at about 15°C to reflux for about 0.5—6 hours.

Alternatively, dimethylsulfate may be used in concentrated aqueous alkali such as sodium or potassium hydroxide at about 15°C to about 50°C.

If the methoxy compound is a racemate or is otherwise optically impure it can be resolved to obtain the (+)- and (−)-enantiomers through preferential crystallization of the diastereomeric salts produced by treatment with one enantiomer of an optically active organic acid such as di-p-tolyltartaric acid or tartaric acid followed by isolation of the optically enriched free base components.

The 9-methoxy compound of this invention is converted to the corresponding hydroxy analog by a variety of reagents and conditions. These include pyridine hydrochloride at about 150 to 250°C for about 3 to 8 hours; boron tribromide or aluminum chloride in an inert solvent such as petroleum ether, toluene, hexane, methylene chloride or tetrachloroethane between room temperature and reflux for 3 to about 8 hours; trimethylsilyl iodide at about 0 to 50°C for about 2 to 8 hours followed by simple acid hydrolysis; sodium cyanide in dimethylsulfoxide at about 150 to 200°C for 3 to about 8 hours; sodium benzylselenolate in refluxing dimethylformamide for ½ to about 3 hours; an alkali metal alkoxide such as lithium thiomethoxide, sodium thioethoxide or lithium thiopropoxide at about 100 to 150°C for 18 to 48 hours; lithium iodide in a basic solvent such as 2,4,6-collidine at about 150 to 200°C for about 6 to 12 hours; sodium p-thiocresolate hexamethylphosphoric triamide in refluxing toluene until the reaction is complete; thioethoxide in dimethylformamide at room temperature to about 100° for 3 to about 12 hours; boron tribromide/sodium iodide/15-crown-5 in dry methylene chloride at about −50 to −10°C for 2 to 5 hours; boron trichloride or tribromide and dimethylsulfide in refluxing 1,2-dichloroethane; and refluxing 48% HBr.

The following Examples 15—22 are examples of alternate processes for the preparation of (+)-*trans*-1a,2,3,4a,5,6-hexahydro-9-methoxy-4-propyl-4H-naphth[1,2,b]-1,4-oxazine hydrochloride. Employing the same reactions described in these examples but using (+)- or (−)- optically active starting materials there are obtained the (+)- or (−)- products. Alternatively, the racemic products are resolved into the (+)- or (−)- products. The corresponding 9-hydroxy compounds are readily available by de-etherification.

Example 15

*Step A:* Preparation of *trans*-1a,2,3,4a,5,6-Hexahydro-9-methoxy-4-propionyl-4H-naphth[1,2-b]-1,4-oxazine hydrochloride

An ethyl acetate (100 ml) solution of trans-1a,2,3,4a-5,6-hexahydro-9-methoxy-4H naphth[1,2-b)-1,4-oxazine (2.0 g, 0.01 mole) is treated with 25 ml, of saturated $Na_2CO_3$ solution followed by 1 ml of propionyl chloride and the resulting mixture is stirred at room temperature for 1 hr. The ethyl acetate layer is separated, washed with brine, dried over $Na_2SO_4$ and then concentrated *in vacuo* and the residue is used directly in the next step.

*Step B:* Preparation of *trans*-1a,2,3,4a,5,6-Hexahydro-9-methoxy-4-propyl-4H-naphth [1,2-b]-1,4-oxazine hydrochloride

The residue from Step A is taken up in THF (40 ml) and added dropwise to a suspension of LAH (300 mg) in THF (40 ml). The reaction mixture is heated at reflux for 1 hr, cooled in ice and excess LAH destroyed with isopropanol. The mixture is treated with methylene chloride (75 ml), saturated $Na_2SO_4$ solution (2 ml), and then filtered (supercell). The filtrate is concentrated *in vacuo* and the residue taken up in ether followed by addition of several ml of ethanolic hydrochloric acid affords the subject compound as a white solid.

Example 16

Preparation of *trans*-1a,2,3,4a,5,6-Hexahdro-9-methoxy-4-propyl-4H-naphth [1,2-b]-1,4-oxazine hydrochloride

A mixture of *trans*-1a,2,3,4a,5,6-hexahydro-9-methoxy-4H-naphth [1,2-b)-1,4-oxazine (2.0 g, 0.01 mole), propionaldehyde (0.6 g, 0.01 mole), 10% Pd/C catalyst (0.5 g) in ethanol (75 ml) is placed on a Parr apparatus and hydrogenated for 5 hrs. The reaction is removed, filtered, and the solvent removed *in vacuo.* The residue is dissolved in ether and treated with ethanolic HCl to afford the subject compound as a white solid.

Example 17

Preparation of *trans*-1a,2,3,4a,5,6-Hexahydro-9-methoxy-4-propyl-4H-naphth [1,2-b]-1,4 oxazine hydrochloride

A methanol solution of *trans*-1a,2,3,4a,5,6-hexahydro-9-methoxy-4H-naphth[1,2-b)-1,4-oxazine (2.0 g, 0.01 mole) and propionaldehyde (0.6 g, 0.01 mole) is stirred mechanically as a solution of sodium cyanoborohydride (0.6 g, 0.01 mole) in methanol is added dropwise. After 1 hr the reaction mixture is poured into water (200 ml) and then extracted with ether (2 × 50 ml). The ether layer is washed with brine, dried over $Na_2SO_4$, and filtered. Addition of ethanolic HCl affords the subject product.

Example 18

*Step A:* Preparation of trans-2-propylamino-7-methoxy-1,2,3,4-tetrahydronaphthalen-1-ol

A solution of trans-2-propionamido-7-methoxy-1,2,3,4-tetrahydronaphthalen-1-ol (2.49 g, 0.01 mole) in the THF (75 ml) is added dropwise to a stirred suspension of LAH (1.2 g) in THF (40 ml) and then the mixture is heated at reflux for 1 hr. After cooling excess LAH is destroyed by the addition of isopropanol, 75 ml of methylene chloride is added, followed by 25 ml of saturated $Na_2SO_4$ solution. This mixture is filtered (supercell), and dried over $Na_2SO_4$. Evaporation of the solvent affords the subject compound.

*Step B:* Preparation of *trans*-1a,2,3,4a,5,6-Hexahydro-9-methoxy-4-propyl-4H naphth[1,2-b]-4H-oxazine hydrochloride

A mixture of trans-2-propylamino-7-methoxy-1,2,3,4-tetrahydronaphthalen-1-ol (2.3 g, 0.01 mole), powdered sodium hydroxide (1.6 g, 0.04 mole), 1,2-dibromoethane (7.52 g, 0.04 mole), methyl tricaprylyl ammonium chloride (0.41 g, 0.01 mole), acetonitrile (32 ml) and dichloromethane (48 ml) is stirred at 25—30° for 24 hr, and then powdered sodium hydroxide (0.4 g, 0.01 mole) is added and stirring is continued for an additional 2 hrs. The mixture is filtered and the residue washed with ether. The filtrate is concentrated *in vacuo*, the resulting residue is dissolved in ether and treated with ethanolic HCl to afford the subject compound.

Example 19

*Step A:* Preparation of *trans*-2-(N-propyl)ethanolamino-7-methoxy-1,2,3,4-tretrahydronaphthalen-1-ol

A solution of ethylene oxide (0.66 g, 0.015 mole) in THF (20 ml) is added to a stirred solution of *trans*-2-propylamino-7-methoxy-1,2,3,4-tetrahydronaphthalen-1-ol (2.49 g, 0.01 mole) in THF (75 ml) and stirring is continued for 24 hrs at room temperature. The solvent is removed *in vacuo* and the residue purified by chromatography to afford the subject compound.

*Step B:* Preparation of trans-2-(N-propyl-N-p-toluenesulfonyloxyethylamino)-7-methoxy-1,2,3,4-tetrahydronaphthalen-1-ol

A mixture containing *trans*-2-(N-propyl)ethanolamino-7-methoxy-1,2,3,4-tetrahydronaphthalen-1-ol (2.8 g, 0.01 mole), (Step A), p-toluenesulfonylchloride (1.9 g, 0.01 mole), 4-dimethylaminopyridine (100 mg) in methylene chloride is stirred at room temperature for 4 hrs. The reaction mixture is washed with brine, dried over $Na_2SO_4$, and then concentrated *in vacuo.*

*Step C:* Preparation of *trans*-1a,2,3,4a,5,6-Hexahydro-9-methoxy-4-propyl-4H-napth [1,2-b]-1,4-oxazine hydrochloride

The residue from Step B is dissolved in a mixture of THF (25 ml), $CH_3CN$ (20 ml), and ethanol (1 ml) and added dropwise to a suspension of NaH (0.48 g, 0.01 mole −50% mineral oil suspension) in THF (25 ml). After stirring for 2 hrs at room temperature the reaction mixture is poured carefully into water and then extracted with ethyl acetate. The ethyl acetate layer is washed with brine, dried over $Na_2SO_4$ and concentrated *in vacuo.* The residue is dissolved in ether and treated with ethanolic HCl to afford the subject compound.

Example 20

Preparation of *trans*-1a,2,3,4a,5,6-Hexahydro-9-methoxy-4-propyl-4H-napth [1,2-b]-1,4-oxazine hydrochloride

To a solution of *trans*-2-(N-propyl)ethanalamino-7-methoxy-1,2,3,4-tetrahydronaphthalen-1-ol (2.9 g, 0.011 mole) (Step A, Example 19) and triphenylphospine (4.98 g, 0.019 mole) in THF (60 ml) is added diethyl azodicarboxylate (2.61 g, 0.015 mole) at 10°C. The mixture is stirred at 10° for 4 hrs. and then placed in the refrigerator for 18 hrs. The solvent is removed and the residue stirred with dichloromethane (60 ml) and $K_2CO_3$ for 2 hrs. The mixture is filtered and the filtrate chromatographed over silica gel. The proper fractions are collected, concentrated, the residue taken up in ether and ethanolic HCl added to afford the title compound.

Example 21

Preparation of *trans*-1a,2,3,4a,5,6-Hexahydro-9-methoxy-4-propyl-4H-naphth[1,2-b]-1,4-oxazine hydrochloride

An ethanolic solution of *trans*-1a,2,3,4a,5,6-hexahydro-9-methoxy-4-allyl-4H-naphth[1,2-b)-1,4-oxazine

(2.59 g, 0.01 mole) containing 0.75 g of 10% Pd/C catalyst is placed on a Parr apparatus and hydrogenated until the proper amount of hydrogen is absorbed. The reaction mixture is removed from the Parr, filtered, and the solvent removed *in vacuo.* The residue is dissolved in ether and treated with ethanolic HCl to afford the subject compound.

Example 22

Preparation of *trans*-1a,2,3,4a,5,6-Hexahydro-9-methoxy-4-propyl-4H-napth[1,2-b]-1,4-oxazine hydrochloride

To a suspension of NaH (0.48 g, 0.01 mole, 50% mineral oil suspension) in THF (25 ml) is added a solution of trans-1a,2,3,4a,5,6-hexahydro-9-hydroxy-4-propyl-4H-naphth[1,2-b]-1,4-oxazine (2.4 g, 0.01 mole) in THF (100 ml) over a period of 0.5 hr. When evolution of hydrogen stops the reaction mixture is cooled in ice and 1.4 g of methyl iodide is added. The reaction is stirred at room temperature for 4 hrs and then 1.0 g of additional methyl iodide added. After an additional 2 hrs a few drops of ethanol are added and then the solvent is removed *in vacuo*. The residue is purified by chromatography ($SiO_2$-solvent $CH_2Cl_2$-acetone 9:1). The proper fractions are collected and concentrated. The residue is dissolved in ether and ethanolic HCl added to afford the subject compound.

Example 23

Resolution of *trans*-1a,2,3,4a,5,6-Hexahydro-9-methoxy-4-propyl-4H-naphth[1,2-b]-1,4-oxazine hydrochloride

To 27.0 g (0.10 mole) of *trans*-1a,2,3,4,5,6-hexahydro-9-methoxy-4-propyl-4H-naphth[1,2-b)-1,4-oxazine was added 41.6 g (0.10 mole) of di-p-toluoyl-*d*-tartaric acid (from natural tartaric acid) in 650 ml of 95% ethanol, and the mixture was heated to produce a solution. After cooling and seeding, the mixture was allowed to stand overnight at room temperature after which the salt was collected by suction filtration to give 29.2 g of partially resolved material. Recrystallization from 300 ml of 95% ethanol gave 23.6 g of (−)-salt; $[\alpha]_D^{25}$ −109.3° (pyridine).

The mother liquor from the first crystallization of the di-p-toluoyl-*d*-tartaric acid salt from above was concentrated, and the residue was partitioned between saturated aqueous $NaHCO_3$ and $CH_2Cl_2$. After drying ($Na_2SO_4$) and concentration, the residue was combined with 23.2 g (0.058 mole) of di-p-toluoyl-*l*-tartaric acid (from unnatural tartaric acid) and the mixture was crystallized from 350 ml of 95% ethanol. After cooling and seeding, the mixture was allowed to stand overnight at room temperature after which filtering gave 11.0 g of (+)-salt; $[\alpha]_D^{25}$ +97.22° (pyridine).

The (+)- and (−)-salts from above were each partitioned between saturated aqueous $NaHCO_3$ and $CH_2Cl_2$. After drying ($Na_2SO_4$) and concentrating, each of the residual free bases was recrystallized from 30—60°C b.p. petroleum ether by cooling to −20°C. Filtration of each gave materials having the following properties:

(−)-"compound"; mp 45—47°C; $[\alpha]_D^{25}$ −62.7°
(c, 0.69, $CH_3OH$);
(+)-"compound"; mp 44—46°C; $[\alpha]_D^{25}$ +56.6°
(c, 0.70, $CH_3OH$);

Example 24

(+)-*trans*-1a,2,3,4a,5,6-Hexahydro-9-methoxy-4-propyl-4H-naphth[1,2-b]-1,4-oxazine via resolution

To 13.0 g (0.05 mole) of racemic *trans*-1a,2,3,4a,5,6-hexahydro-9-methoxy-4-propyl-4H-naphth[1,2-b]-1,4-oxazine in 500 ml of 95% ethanol was added 20.2 g (0.05 mole) of di-p-touoyl-*l*-tartaric acid (from unnatural tartaric acid), and the mixture was heated to produce a solution. After cooling and seeding, the mixture was allowed to stand overnight at room temperature and the salt was collected by suction filtration to give 9.1 g of partially resolved material; m.p. 170—172°C' $[\alpha]_D^{25}$ +103.9° (C, 0.59, pyridine). This salt was recrystallized from 95 ml of 95% ethanol to give 6.8 g of salt which was partitioned between saturated aqueous $NaHCO_3$ and $CH_2Cl_2$. After drying ($Na_2SO_4$) and concentrating the $CH_2Cl_2$ solution, 2.7 g (0.01 mole) of free base was isolated. This material was mixed with 1.5 g (0.01 m) of *d*-tartaric acid in ethanol, and the solution was heated and concentrated to about 30 ml. After standing for several days, 2.8 g of salt were collected by filtration. This material was resuspended in 75 ml of ethanol with heating and allowed to cool slowly. The salt was filtered and the mother liquor concentrated to a residue which was partitioned between saturated aqueous $NaHCO_3$ and $CH_2Cl_2$. After drying ($Na_2SO_4$) and concentrating, the residue was recrystallized from 30—60°C b.p. petroleum ether by cooling to about −20°C. Filtration and drying gave 650 mg of (+)="title compound"; m.p. 46—47°C; $[\alpha]_D^{25}$ +61.3° (c, 0.65, $CH_3OH$).

**Claims for the Contracting States: BE CH DE FR GB IT LI LU NL SE**

1. Compounds of structural formula I:

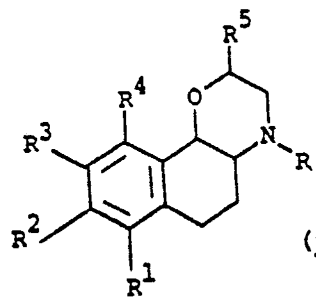

(trans)

with positive, negative or zero optical activity, or salts thereof, wherein
R is
- a) hydrogen,
- b) $C_{1-4}$ alkyl,
- c) $C_{2-5}$ alkenyl,
- d) phenyl-$C_{1-4}$ alkyl; and

$R^1$, $R^2$, $R^3$ and $R^4$ are independently:
- a) hydrogen,
- b) $C_{1-4}$ alkyl,
- c) halo,
- d) $OR^6$ wherein $R^6$ is
  - 1) hydrogen
  - 2) $C_{1-3}$ alkyl,
  - 3) phenyl-$C_{1-3}$ alkyl,
  - 4) $-\underset{\underset{O}{\|}}{C}-R^7$ wherein $R^7$ is
    - i) $C_{1-6}$ alkyl
    - ii) $C_{3-6}$ cycloalkyl
    - iii) phenyl-$C_{1-3}$ alkyl, wherein the phenyl group is unsubstituted or substituted with one or more halo, $C_{1-3}$ alkyl, or $C_{1-3}$ alkoxy
    - iv) pyridyl-$C_{1-3}$ alkyl, or furyl-$C_{1-3}$ alkyl
    - v) phenyl, either unsubstituted or substituted with one or more halo, $C_{1-3}$ alkyl, or $C_{1-3}$ alkoxy,
    - vi) pyridyl or furyl and
    - vii) $-NR^8R^9$, wherein $R^8$ and $R^9$ are independently hydrogen or $C_{1-3}$ alkyl, or $R^8$ and $R^9$ may be joined together to form a heterocyclic piperidinyl, morpholinyl, piperazinyl or N—$C_{1-3}$ alkylpiperazinyl ring with the nitrogen to which they are attached,
- e) two adjacent groups selected from $R^1$, $R^2$, $R^3$ and $R^4$ form together an alkylene-dioxy group and

$R^5$ is hydrogen, $C_{1-3}$ alkyl or phenyl.

2. A compound of formula I or a salt thereof wherein:
R and $R^5$ have the same meaning as in claim 1 and
$R^1$, $R^2$, $R^3$ and $R^4$ are independently:
- a) hydrogen,
- b) $C_{1-4}$ alkyl,
- c) halo
- d) $OR^6$ wherein $R^6$ is
  - 1) hydrogen
  - 2) $C_{1-3}$ alkyl,
  - 3) phenyl-$C_{1-3}$ alkyl,
  - 4) $-\underset{\underset{O}{\|}}{C}-R^7$

wherein $R^7$ is as defined in i) through vi) in claim 1 or
vii) $-NR^8R^9$, wherein $R^8$ and $R^9$ are independently hydrogen or $C_{1-3}$ alky, or $R^8$ and $R^9$ may be joined together to form with the nitrogen to which they are attached a heterocycle selected from piperidinyl, morpholinyl, piperazinyl and N—$C_{1-3}$ alkylpiperazinyl
- e) two adjacent groups selected from $R^1$, $R^2$, $R^3$ and $R^4$ form together a methylene-dioxy group.

3. A compound of claim 1 or 2 having the structural formula Ia

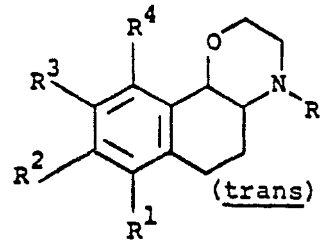

Ia

with positive, negative or zero optical activity, or salts thereof, wherein: R has the same meaning as in formula I of claim 1 and $R^1$, $R^2$, $R^3$ and $R^4$ are independently

a) hydrogen,
b) $C_{1-4}$ alkyl,
c) halo,
d) $OR^6$ wherein $R^6$ is
  1) hydrogen
  2) $C_{1-3}$ alkyl or
  3) phenyl-$C_{1-3}$ alkyl,
  4) $—CO—R^7$ or
e) two adjacent groups selected from $R^1$, $R^2$, $R^3$ and $R^4$ form together a methylenedioxy group and preferably a compound of formula IA wherein:
R is n-propyl,
$R^3$ is hydroxy or methoxy and
$R^1$, $R^2$ and $R^4$ are hydrogen.

4. A process for the preparation of a compound of structural formula Ia:

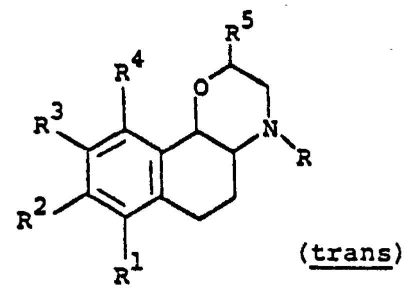

I

according to claim 1 with positive, negative or zero optical activity, or salts of the compound of formula I wherein R, $R^1$, $R^2$, $R^3$, $R^4$ and $R^5$ have the same meaning as in claim 1, characterized in that compounds of formula II:

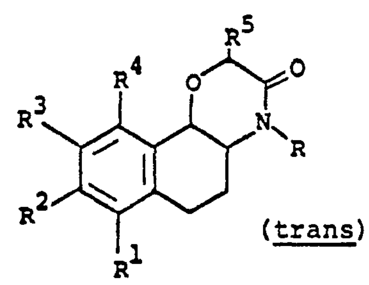

II

with positive, negative or zero optical activity, wherein the radicals R, $R^1$, $R^2$, $R^3$, $R^4$ and $R^5$ have the same meaning as in formula I are reduced, followed, if desired wherein R is hydrogen, by alkylation or alkenylation to provide the compound wherein R is $C_{1-4}$ alkyl, $C_{2-5}$ alkenyl, or phenyl-$C_{1-4}$ alkyl and/or followed, if desired, when one or more of $R^1$, $R^2$, $R^3$ and $R^4$ is $C_{1-3}$ alkoxy, phenyl-$C_{1-3}$ alkoxy, or two are adjacent groups selected from $R^1$, $R^2$, $R^3$, $R^4$, taken together are an alkylene-dioxy group, by deetherification to provide the compound wherein one or more of $R^1$, $R^2$, $R^3$ and $R^4$ is hydroxy; and/or followed, if desired by acylation or carbamylation with an anhydride of formula $(R^7CO)_2O$ or acid chloride of structure $R^7COCl$ to produce the compound wherein one or more of $R^1$, $R^2$, $R^3$ and $R^4$ is

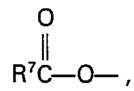

and, that thereafter the compounds of formula I or salts thereof are isolated.

5. Process according to claim 4 wherein compounds of claim 2 or salts thereof are prepared.

6. Process according to claim 4 or 5, characterized in that compounds of structural formula Ia

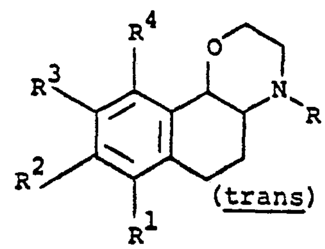

Ia

according to claim 3 with positive, negative or zero optical activity, or salts thereof, are prepared, wherein: R, $R^1$, $R^2$, $R^3$ and $R^4$ have the same meaning as in claim 3, characterized in that the compounds of structural formula IIa

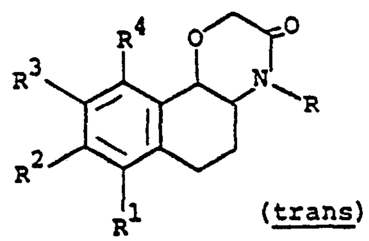

IIa

with positive, negative or zero optical activity, is reduced, wherein: R, $R^1$, $R^2$, $R^3$ and $R^4$ have the same meaning as in formula Ia, followed, if desired wherein R is hydrogen, by alkylation to provide the compound wherein R is $C_{1-4}$ alkyl, $C_{2-5}$ alkenyl, or phenyl-$C_{1-4}$ alkyl, and/or followed, if desired, when one or more of $R^1$, $R^2$, $R^3$ and $R^4$ is $C_{1-3}$ alkoxy, phenyl-$C_{1-3}$ alkoxy, or R groups taken together are methylenedioxy, by deetherification to provide the compound wherein one or more of $R^1$, $R^2$, $R^3$ and $R^4$ is hydroxy and wherein thereafter the compounds of formula Ia or a salt thereof are isolated.

7. Process for the preparation of a compound of structural formula Ib

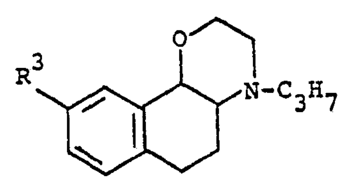

Ib

or a salt of the compound of formula Ib, wherein $R^3$ is —OH or —$OCH_3$, characterized in that said process comprises:

a) reduction of the 4-propionyl group a compound of structural formula:

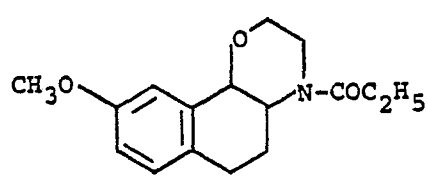

with zero or positive optical activity followed, if desired, by optical resolution to the positive enantiomer followed, if desired, by demethylation to produce the compound wherein $R^3$ is —OH;

b) reductive alkylation of a 4-hydrogen compound of structural formula:

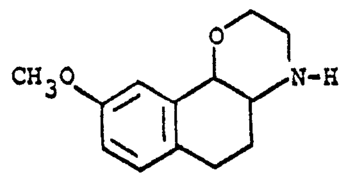

with zero or positive optical activity by treatment with propionaldehyde and a reducing agent followed, if desired, by optical resolution to the positive enantiomer followed, if desired, by demethylation to produce the compound wherein $R^3$ is OH;

c) double condensation tetrahydro-oxazine ring formation by treatment of a compound of structural formula:

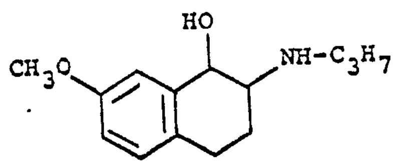

with zero or positive optical activity with a compound of structural formula:

$$X'—CH_2CH_2—X^2$$

wherein X' and $X^2$ are the same or different and represent bromo, iodo, benzenoid aromatic sulfonyloxy, or $C_{1-3}$ alkanesulfonyloxy, followed if desired by optical resolution to the positive enantiomer, followed if desired by demethylation to produce the compound wherein $R^3$ is OH;

d) closure of the tetrahydro-oxazine ring by treatment of a compound of structural formula:

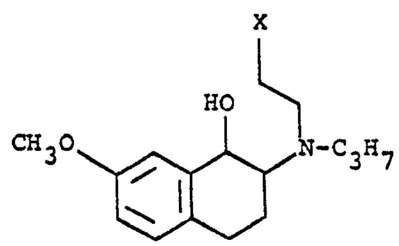

with zero or positive optical activity wherein X is bromo, iodo, benzenoid arylsulfonyloxy, $C_{1-3}$ alkanesulfonyloxy or hydroxy, with a condensing agent, followed if desired by optical resolution to the positive enantiomer, followed if desired by demethylation to produce the compound wherein $R^3$ is OH;

e) reduction of a 4-allyl group by treatment of a compound of structural formula:

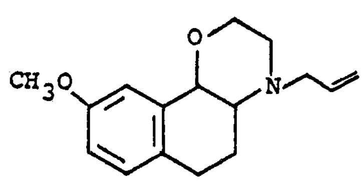

with zero or positive optical activity with a reducing agent followed if desired by optical resolution to the positive enantiomer, followed if desired by demethylation to produce the compound wherein $R^3$ is OH;

f) methylation of a compound of structural formula:

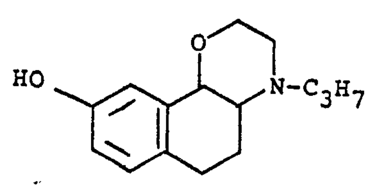

with zero or positive optical activity by treatment with a methylating agent, to produce the compound

wherein $R^3$ is $-OCH_3$ followed if desired by resolution to the positive enantiomer;

g) reduction of a compound of structural formula:

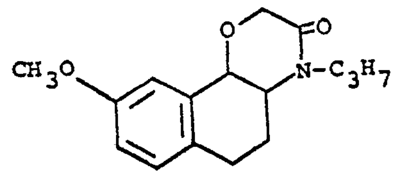

with zero or positive optical rotation by treatment with a reducing agent, followed if desired by optical resolution to the positive enantiomer, followed if desired by demethylation to produce the compound wherein $R^3$ is OH;

and furthermore isolation of the compound of formula Ib or the salt thereof.

8. A pharmaceutical formulation for the treatment of Parkinsonism, depressions, or hypertension which comprises as active ingredient a compound of structural formula I:

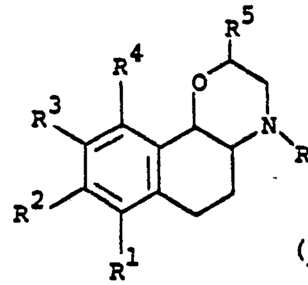

(trans) I

with positive or zero optical activity, or a pharmaceutically acceptable salt thereof, wwherein: R, $R^1$, $R^2$, $R^3$, $R^4$ and $R^5$ have the same meaning as in claim 1.

9. A pharmaceutical formulation according to claim 8, characterized in that it comprises as active ingredient a compound of formula I according to claim 2 or a pharmaceutically acceptable salt of said compound of formula I and, furthermore, a pharmaceutical carrier material.

10. A pharmaceutical formulation according to claim 8 or 9, characterized in that it comprises as pharmaceutically effective compound a compound of formula Ia according to claim 3 or a pharmaceutically acceptable salt thereof and, furthermore, a pharmaceutical carrier material.

**Claims for the Contracting State: AT**

1 A process for the preparation of a compound of structural formula I:

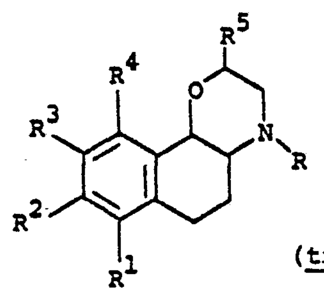

(trans) I

with positive, negative or zero optical activity, and salts thereof, wherein:

R is

a) hydrogen,

b) $C_{1-4}$ alkyl,

c) $C_{2-5}$ alkenyl,

d) phenyl-$C_{1-4}$ alkyl; and

$R^1$, $R^2$, $R^3$ and $R^4$ are independently:

a) hydrogen

b) $C_{1-4}$ alkyl,

c) halo,

d) $OR^6$ wherein $R^6$ is
1) hydrogen
2) $C_{1-3}$ alkyl,
3) phenyl-$C_{1-3}$ alkyl,
4) —C—$R^7$
||
O

wherein $R^7$ is
i) $C_{1-6}$ alkyl
ii) $C_{3-6}$ cycloalkyl
iii) phenyl-$C_{1-3}$ alkyl, wherein the phenyl group is unsubstituted or substituted with one or more halo, $C_{1-3}$ alkyl, or $C_{1-3}$ alkoxy
iv) pyridyl-$C_{1-3}$ alkyl or furyl-$C_{1-3}$ alkyl,
v) phenyl, either unsubstituted or substituted with one or more halo, $C_{1-3}$ alkyl, or $C_{1-3}$ alkoxy,
vi) pyridyl or furyl and
vii) —$NR^8R^9$, wherein $R^8$ and $R^9$ are independently hydrogen or $C_{1-3}$ alkyl, or $R^8$ and $R^9$ may be joined together to form a heterocycle with the nitrogen to which they are attached, the heterocycle being selected from piperidinyl, moropholinyl, piperazinyl or N—$C_{1-3}$ -alkylpiperazinyl,
e) two adjacent groups selected from $R^1$, $R^2$, $R^3$ and $R^4$ form together an alkylene-dioxy group and $R^5$ is hydrogen, $C_{1-3}$ alkyl or phenyl, which comprises the reduction of a compound of structural formula II:

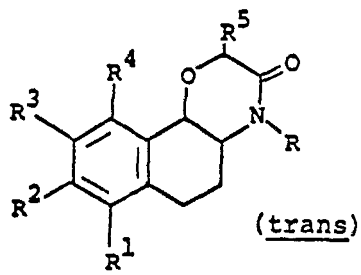

(trans)

II

with positive, negative or zero optical activity followed, if desired wherein R is hydrogen, by alkylation or alkenylation to provide the compound wherein R is $C_{1-4}$ alkyl, $C_{2-5}$ alkenyl, or phenyl-$C_{1-4}$ alkyl and/or followed, if desired, when one or more of $R^1$, $R^2$, $R^3$ and $R^4$ is $C_{1-3}$ alkoxy, phenyl-$C_{1-3}$ alkoxy, or two are adjacent groups selected from $R^1$, $R^2$, $R^3$, $R^4$, taken together are an alkylene-dioxy group, by deetherification to provide the compound wherein one or more of $R^1$, $R^2$, $R^3$ and $R^4$ is hydroxy; and/or followed, if desired by acylation or carbamylation with an anhydride of formula $(R^7CO)_2O$ or acid chloride of structure $R^7COCl$ to produce the compound wherein one or more of $R^1$, $R^2$, $R^3$ and $R^4$ is

O
||
$R^7$C—O—,

and, thereafter, isolating the compound of formula I or a salt thereof.

2. Process according to patent claim 1 characterized in that compounds of formula I or salts thereof are prepared wherein R and $R^5$ have the same meaning as in claim 1 and $R^1$, $R^2$, $R^3$ and $R^4$ are independently:
a) hydrogen,
b) $C_{1-4}$ alkyl,
c) halo,
d) $OR^6$ wherein $R^6$ is
1) hydrogen
2) $C_{1-3}$ alkyl,
3) phenyl-$C_{1-3}$ alkyl,
4) —C—$R^7$
||
O

wherein $R^7$ is as defined in i) through vi) in claim 1 or
vii) —$NR^8R^9$, wherein $R^8$ and $R^9$ are independently hydrogen or $C_{1-3}$ alkyl, or $R^8$ and $R^9$ may be joined together to form with the nitrogen to which they are attached a hetereocycle selected from piperidinyl, morpholinyl, piperazinyl and N—$C_{1-3}$ alkylpiperazinyl
e) two adjacent groups selected from $R^1$, $R^2$, $R^3$ and $R^4$ form together a methylene-dioxy group.

3. Process according to patent claim 1 or 2, characterized in that compounds of structural formula Ia:

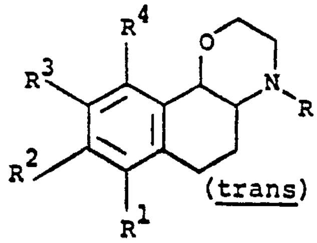

Ia

with positive, negative or zero optical activity, or salts thereof are prepared wherein R has the same meaning as in formula I and $R^1$, $R^2$, $R^3$ nad $R^4$ are independently:

a) hydrogen,
b) $C_{1-4}$ alkyl,
c) halo,
d) $OR^6$ wherein $R^6$ is
  1) hydrogen
  2) $C_{1-3}$ alky or
  3) phenyl-$C_{1-3}$ alkyl,
  4) $-COR^7$ or

e) two adjacent groups selected from $R^1$, $R^2$, $R^3$ and $R^4$ form together a methylene-dioxy group and preferably compounds of formula Ia are prepared wherein

R is n-propyl,
$R^3$ is hydroxy or methoxy and
$R^1$, $R^2$ and $R^4$ are hydrogen, in which process a compound of structural formula IIa

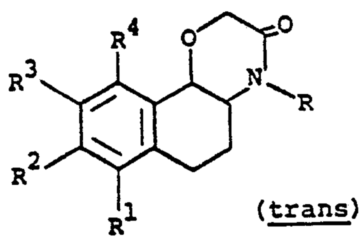

IIa

with positive, negative or zero optical activity, is reduced, followed, if desired wherein R is hydrogen, by alkylation to provide the compound wherein R is $C_{1-4}$ alkyl, $C_{2-5}$ alkenyl, or phenyl-$C_{1-4}$ alkyl, and/or followed, if desired, when one or more of $R^1$, $R^2$, $R^3$ and $R^4$ is $C_{1-3}$ alkoxy, phenyl-$C_{1-3}$ alkoxy, or R groups taken together are methylene-dioxy, by deetherification to provide the compound wherein one or more of $R^1$, $R^2$, $R^3$ and $R^4$ is hydroxy and wherein thereafter the compounds of formula Ia or a salt thereof are isolated.

4. A process for the preparation of a compound of structural formula Ib

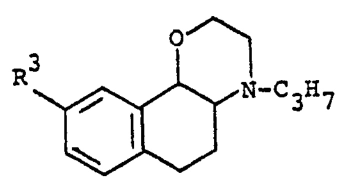

Ib

with zero or positive optical activity wherein $R^3$ is $-OH$ or $-OCH_3$ or a salt of the compounds of formula Ib which comprises:

a) reduction of the 4-propionyl group a compound of structural formula:

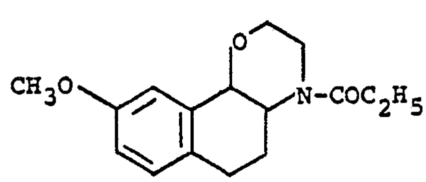

with zero or positive optical activity followed, if desired, by optical resolution to the positive enantiomer folllowed, if desired, by demethylation to produce the compound wherein $R^3$ is $-OH$;

b) reductive alkylation of a 4-hydrogen compound of structural formula:

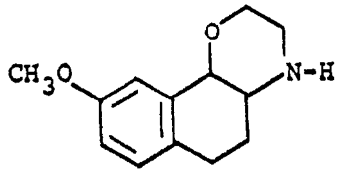

with zero or positive optical activity by treatment with propionaldehyde and a reducing agent followed, if desired by optical resolution to the positive enantiomer followed, if desired, by demethylation to produce the compound wherein $R^3$ is OH;

c) double condensation of tetrahydro-oxazine ring formation by treatment of a compound of structural formula:

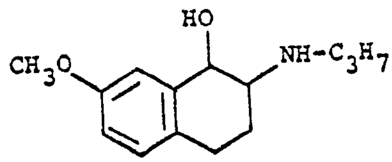

with zero or positive optical activity with a compound of structural formula:

$$X'—CH_2CH_2—X^2$$

wherein X' and $X^2$ are the same or different and represent bromo, iodo, benzenoid aromatic sulfonyloxy, or $C_{1-3}$ alkanesulfonyloxy, followed if desired by optical resolution to the positive enantiomer, followed if desired by demethylation to produce the compound wherein $R^3$ is OH;

(d) closure of the tetrahydro-oxazine ring by treatment of a compound of structural formula:

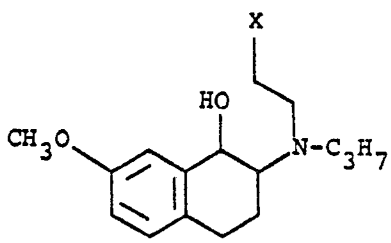

with zero or positive optical activity wherein X is bromo, iodo, benzenoid arylsulfonyloxy, $C_{1-3}$ alkanesulfonyloxy or hydroxy, with a condensing agent, followed if desired by optical resolution to the positive enantiomer, followed, if desired by demethylation to produce the compound wherein $R^3$ is OH;

e) reduction of a 4-allyl group by treatment of a compound of structural formula:

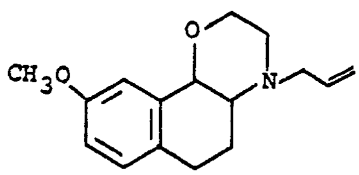

with zero or positive optical activity with a reducing agent followed if desired by optical resolution to the positive enantiomer, followed if desired by demethylation to produce the compound wherein $R^3$ is OH;

f) methylation of a compound of structural formula:

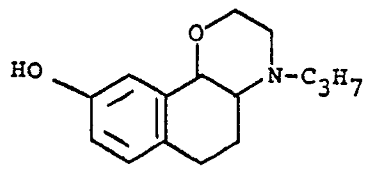

with zero or positive optical activity by treatment with a methylating agent, to produce the compound wherein $R^3$ is —$OCH_3$ followed if desired by resolution to the positive enantiomer;

g) reduction of a compound of structural formula:

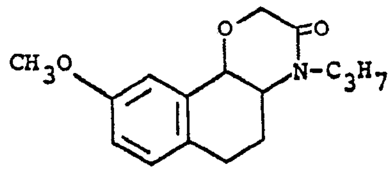

with zero or positive optical rotation by treatment with a reducing agent, followed if desired by optical resolution to the positive enantiomer, followed if desired by demethylation to produce the compound wherein $R^3$ is OH;
and furthermore isolation of the compound of formula Ib or the salt thereof.

5. Process for the preparation of a pharmaceutical composition for the treatment of Parkinsonism, depressions or hypertension characterized in that a pharmaceutical composition is formulated which contains as pharmaceutically active ingredient at least one compound of structural formula I:

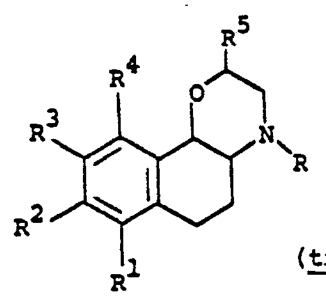

I

(trans)

with positive or zero optical activity, prepared according to the process of claim 1, wherein: R, $R^1$, $R^2$, $R^3$, $R^4$ and $R^5$ have the same meaning as in claim 1 or a pharmaceutically acceptable salt of the compounds of formula I.

6. Process according to claim 5 characterized in that the pharmaceutical composition is formulated using as active ingredient a compound of formula I prepared according to the process of patent claim 2 or a pharmaceutically acceptable salt thereof.

7. Process according to patent claim 5 characterized in that a pharmaceutical composition is prepared by formulating as active ingredient a compound of formula I prepared according to the process of claim 3 or a pharmaceutically acceptable salt thereof.

8. Process according to claim 5 characterized in that the pharmaceutical composition is formulated by incorporating into it as active ingredient a compound of formula Ib prepared according to the process of claim 5 or a pharmaceutically acceptable salt thereof.

9. Process according to one of the patent claims 5—8 characterized in that a pharmaceutical composition is prepared which contains as further component a pharmaceutical carrier material.

**Patentansprüche für die Vertragsstaaten: BE CH DE FR GB IT LI LU NL SE**

1. Verbindungen der Strukturformel I

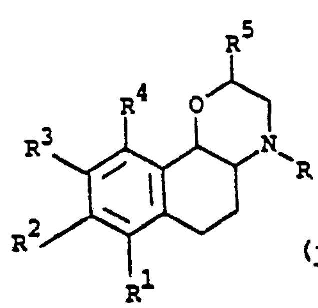

(trans)

mit positiver, negativer oder ohne optische Aktivität, oder ihre Salze, worin:

R a) Wasserstoff,

b) $C_{1-4}$-Alkyl,

c) $C_{2-5}$-Alkenyl,
d) Phenyl-$C_{1-4}$-alkyl ist; und
$R^1$, $R^2$, $R^3$ und $R^4$ unabhängig voneinander
a) Wasserstoff,
b) $C_{1-4}$-Alkyl,
c) Halogen,
d) $OR^6$ sind, worin $R^6$
1) Wasserstoff
2) $C_{1-3}$-Alkyl,
3) Phenyl-$C_{1-3}$-alkyl,
4) $-\underset{\underset{O}{\|}}{C}-R^7$ ist, worin $R^7$
i) $C_{1-6}$-Alkyl,
ii) $C_{3-6}$-Cycloalkyl,
iii) Phenyl-$C_{1-3}$-alkyl, worin die Phenylgruppe unsubstituiert oder mit einem oder mehreren Halogen substituiert ist, $C_{1-3}$-Alkyl oder $C_{1-3}$-Alkoxy,
iv) Pyridyl-$C_{1-3}$-alkyl oder Furyl-$C_{1-3}$-alkyl,
v) Phenyl, entweder unsubstituiert oder mit einem oder mehreren Halogen substituiert, $C_{1-3}$-Alkyl oder $C_{1-3}$-Alkoxy,
vi) Pyridyl oder Furyl und
vii) $-NR^8R^9$ ist, worin $R^8$ und $R^9$ unabhängig voneinander Wasserstoff oder $C_{1-3}$-Alkyl sind oder $R^8$ und $R^9$ mit dem Stickstoff, an welchen sie gebunden sind, unter Bildung eines heterocyclischen Piperidinyl-, Morpholinyl-, Piperazinyl- oder N—$C_{1-3}$-Alkylpiperazinylrings zusammengenommen werden können,
oder
e) zwei aus $R^1$, $R^2$, $R^3$ und $R^4$ ausgewählte benachbarte Gruppen zusammen eine Alkylendioxygruppe bilden, und
$R^5$ Wasserstoff, $C_{1-3}$-Alkyl oder Phenyl ist.

2. Verbindung nach Anspruch 1 oder ein Salz davon, worin
R und $R^5$ die gleiche Bedeutung wie in Anspruch 1 haben
und
$R^1$, $R^2$, $R^3$ und $R^3$ unabhängig voneinander
a) Wasserstoff,
b) $C_{1-4}$-Alkyl,
c) Halogen,
d) $OR^6$ sind, worin $R^6$
1) Wasserstoff
2) $C_{1-3}$-Alkyl,
3) Phenyl-$C_{1-3}$-alkyl,
4) $-\underset{\underset{O}{\|}}{C}-R^7$ ist, worin $R^7$ wie in i) bis vi)
in Anspruch 1 definiert ist oder
vii) $-NR^8R^9$ ist, worin $R^8$ und $R^9$ unabhängig voneinander Wasserstoff oder $C_{1-3}$-Alkyl sind oder $R^8$ und $R^9$ unter Bildung mit dem Stickstoff, an den sie gebunden sind, eines aus Piperidinyl, Morpholinyl, Piperazinyl und N—$C_{1-3}$-Alkylpiperazinyl auswählten Heterocyclus zusammengenommen werden können,
oder
e) zwei aus $R^1$, $R^2$, $R^3$ und $R^4$ ausgewählte benachbarte Gruppen zusammen eine Methylendioxygruppe bilden.

3. Verbindung nach Anspruch 1 oder 2 mit der Strukturformel Ia

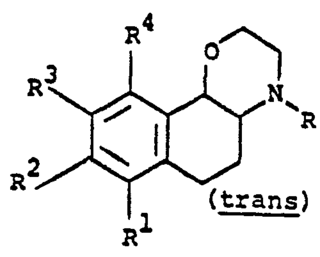

Ia

mit positiver, negativer oder ohne optische Aktivität oder ihre Salze, worin

R die gleiche Bedeutung wie in Formel I in Anspruch 1 aufweist und $R^1$, $R^2$, $R^3$ und $R^4$ unabhängig voneinander

a) Wasserstoff,
b) $C_{1-4}$-Alkyl,
c) Halogen,
d) $OR^6$ sind, worin $R^6$
  1) Wasserstoff,
  2) $C_{1-3}$-Alkyl oder
  3) Phenyl-$C_{1-3}$-Alkyl,
  4) $—CO—R^7$ ist oder
e) zwei aus $R^1$, $R^2$, $R^3$ und $R^4$ ausgewählte benachbarte Gruppen zusammen eine Methylendioxygruppe bilden und

vorzugsweise eine Verbindung der Formel Ia, worin
R n-Propyl ist,
$R^3$ Hydroxy oder Methoxy ist und
$R^1$, $R^2$ und $R^4$ Wasserstoff sind.

4. Verfahren zur herstellung einer Verbindung der Strukturformel Ia

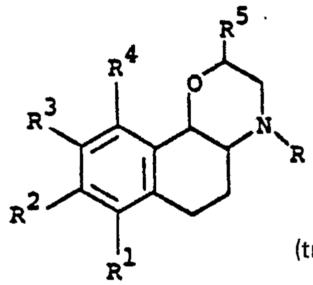

(trans)

I

nach Anspruch 1 mit positiver, negativer oder ohne optische Aktivität, oder Salzender Verbindung der Formel I, worin R, $R^1$, $R^2$, $R^3$, $R^4$ und $R^5$ die gleiche Bedeutung wie in Anspruch 1 haben, dadurch gekennzeichnet, daß Verbindungen der Formel II

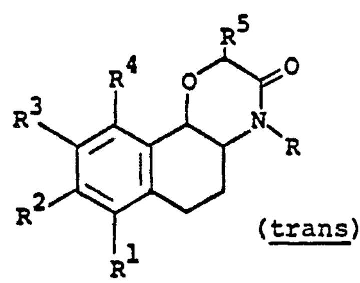

(trans)

II

mit positiver, negativer oder ohne optische Aktivität, worin die Radikale R, $R^1$, $R^2$, $R^3$, $R^4$ und $R^5$ die gleiche Bedeutung wie in Formel I haben, reduziert werden, gefolgt, falls erwünscht, wenn R Wasserstoff ist, von der Alkylierung oder Alkenylierung zur Bereitstellung der Verbindung, worin R $C_{1-4}$-Alkyl, $C_{2-5}$-Alkenyl oder Phenyl-$C_{1-4}$-alkyl ist, und/oder gefolgt, falls erwünscht, wenn ein oder mehrere von $R^1$, $R^2$, $R^3$ und $R^4$ $C_{1-3}$-Alkoxy, Phenyl-$C_{1-3}$-alkoxy sind oder zwei aus $R^1$, $R^2$, $R^3$, $R^4$ ausgewählte Gruppen benachbart sind und zusammengenommen eine Alkylendioxygruppe sind, von der Deetherifizierung zur Bereitstellung der Verbindung, worin ein oder mehrere von $R^1$, $R^2$, $R^3$ und $R^4$ Hydroxy sind; und/oder gefolgt, falls erwünscht, von der Acylierung oder Carbamylierung mit einem Anhydrid der Formel $(R^7CO)_2O$ oder Säurechlorid der Struktur $R^7COCl$ zur Herstellung der Verbindung, worin eines oder mehrere von $R^1$, $R^2$, $R^3$ und $R^4$

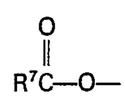

sind, und daß danach die Verbindungen der Formel I oder ihre Salze isoliert werden.

5. Verfahren nach Anspruch 4, worin Verbindungen nach Anspruch 2 oder ihre Salze hergestellt werden.

6. Verfahren nach Anspruch 4 oder 5, dadurch gekennzeichnet, daß Verbindung der Strukturformel Ia

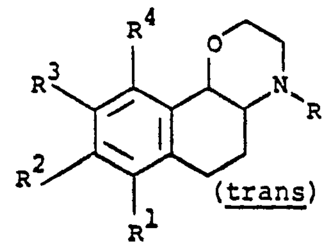

Ia

gemäss Anspruch 3 mit positiver, negativer oder ohne optische Aktivität oder ihre Salze hergestellt werden, worin R, $R^1$, $R^2$, $R^3$ und $R^4$ die gleiche Bedeutung wie in Anspruch 3 haben, dadurch gekennzeichnet, dass die Verbindungen der Strukturformel IIa

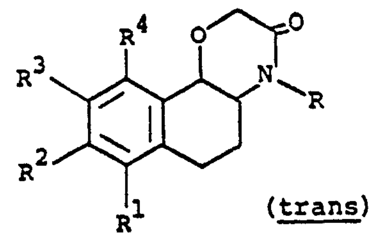

IIa

mit positiver, negativer oder ohne optische Aktivität reduziert werden, worin R, $R^1$, $R^2$, $R^3$ und $R^4$ die gleiche Bedeutung wie in Formel Ia haben, gefolgt, falls erwünscht, wenn R Wasserstoff ist, von der Alkylierung zur Bereitstellung der Verbindung, worin R $C_{1-4}$-Alkyl, $C_{1-5}$-Alkenyl oder Phenyl-$C_{1-4}$-alkyl ist, und/oder gefolgt, falls erwünscht, wenn eines oder mehrere von $R^1$, $R^2$, $R^3$ und $R_1$ $C_{1-3}$-Alkoxy, Phenyl-$C_{1-3}$-alkoxy sind oder R-Gruppen, zusammengenommen, Methylendioxy sind, von der Deetherifizierung unter Bereitstellung der Verbindung, worin eines oder mehrere von $R^1$, $R^2$, $R^3$ und $R^4$ Hydroxy sind und worin danach die Verbindungen der Formel Ia oder ein Salz davon isoliert werden.

7. Verfahren zur Herstellung einer Verbindung der Strukturformel Ib,

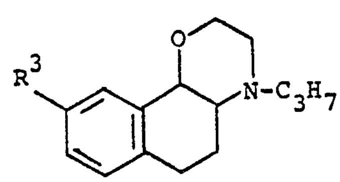

Ib

oder eines Salzes der Verbindung der Formel Ib, worin $R^3$ —OH oder —$OCH_3$ ist, dadurch gekennzeichnet, dass das Verfahren umfasst:

(a) Reduktion der 4-Propionylgruppe einer Verbindung der Strukturformel

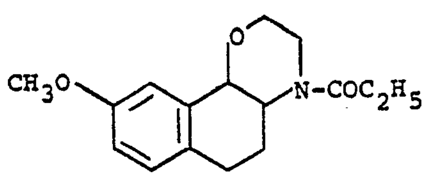

mit null oder positiver optischer Aktivität, gefolgt, falls erwünscht, von der optischen Auflösung zum positiven Enantiomer, gefolgt, falls erwünscht, von der Demethylierung zur Herstellung der Verbindung, worin $R^3$ —OH ist;

(b) reduktive Alkylierung einer 4-Wasserstoff-Verbindung der Strukturformel

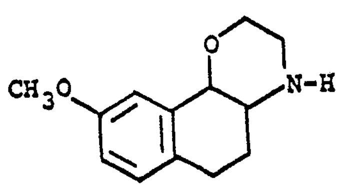

mit null oder positiver optischer Aktivität durch behandlung mit Propionaldehyd und einem

Reduktionsmittel, gefolgt, falls erwünscht, von der optischen Auflösung zum positiven Enantiomer, gefolgt, falls erwünscht, von der Demethylierung zur Herstellung der Verbindung, worin $R^3$ OH ist;
(c) Tetrahydrooxazin-Ringbildung mittels doppelter Kondensation durch Behandlung einer Verbindung der Strukturformel

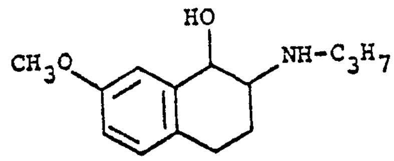

mit null oder positiver optischer Aktivität mit einer Verbindung der Strukturformel

$$X'—CH_2CH_2—X^2$$

worin X' und $X^2$ gleich oder verschieden sind und Brom, Jod, benzolisches aromatisches Sulfonyloxy oder $C_{1-3}$-Alkylsulfonyloxy darstellen, gefolgt, falls erwünscht, von der optischen Auflösung zum positiven Enantiomeren, gefolgt, falls erwünscht, von der Demethylierung zur Herstellung der Verbindung, worin $R^3$ OH ist;
(d) Schliessung des Tetrahydrooxazinrings durch Behandlung einer Verbindung der Strukturformel

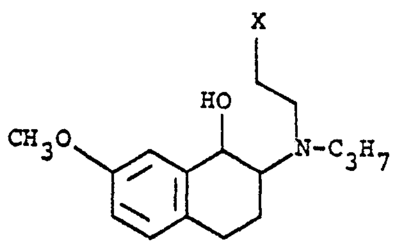

mit null oder positiver optischer Aktivität, worin X Brom, Jod, benzolisches Arylsulfonyloxy, $C_{1-3}$-Alkylsulfonyloxy oder Hydroxy ist, mit einem Kondensationsmittel, gefolgt, falls erwünscht, von der optischen Auflösung zum positiven Enantiomeren, gefolgt, falls erwünscht, von der Demethylierung zur Herstellung der Verbindung, worin $R^3$ OH ist;
(e) Reduktion einer 4-Allylgruppe durch Behandlung einer Verbindung der Strukturformel

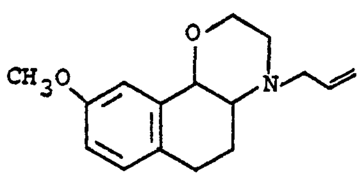

mit null oder positiver optischer Aktivität mit einem Reduktionsmittel, gefolgt, falls erwünscht, von der optischen Auflösung zum positiven Enantiomeren, gefolgt, falls erwünscht, von der Demethylierung zur Herstellung der Verbindung, worin $R^3$ OH ist;
(f) Methylierung einer Verbindung der Strukturformel

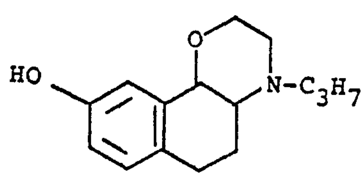

mit null oder positiver optischer Aktivität durch Behandlung mit einem Methylierungsmittel zur Herstellung der Verbindung, worin $R^3$ $OCH_3$ ist, gefolgt, falls erwünscht, von der Auflösung zum positiven Enantiomeren;

(g) Reduktion einer Verbindung der Strukturformel

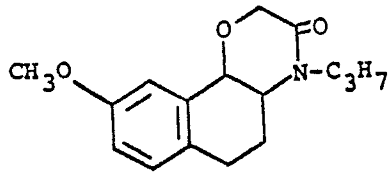

mit null oder positiver optischer Rotation durch Behandlung mit einem Reduktionsmittel, gefolgt, falls erwünscht, von der optischen Auflösung zum positiven Enantiomeren, gefolgt, falls erwünscht, von der Demethylierung zur Herstellung der Verbindung, worin $R^3$ OH ist; und weiterhin Isolierung der Verbindung der Formel Ib oder ihres Salzes.

8. Pharmazeutische Formulierung zur Behandlung von Parkinsonismus, Depressionen oder Bluthochdruck, welche als aktiven bestandteil eine Verbindung der Strukturformel I

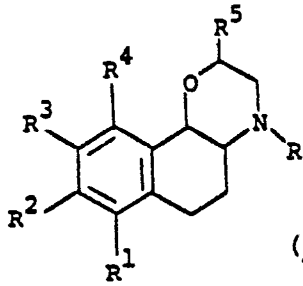

I

(trans)

mit positiver oder ohne optische Aktivität, oder ein pharmazeutisch annehmbares Salz davon enthält, worin R, $R^1$, $R^2$, $R^3$, $R^4$ und $R^5$ die gleiche Bedeutung wie in Anspruch 1 haben.

9. Pharmazeutische Formulierung nach Anspruch 8, dadurch gekennzeichnet, dass sie als aktiven Bestandteil eine Verbindung der Formel I nach Anspruch 2 oder ein pharmazeutisch annehmbares Salz dieser Verbindung der Formel I und, weiterhin, ein pharmazeutisches Trägermaterial enthält.

10. Pharmazeutische Formulierung nach Anspruch 8 oder 9, dadurch gekennzeichnet, dass sie als pharmazeutisch wirksame Verbindung eine Verbindung der Formel Ia nach Anspruch 3 oder ein pharmazeutisch annehmbares Salz davon und, weiterhin, ein pharmazeutisches Trägermaterial enthält.

**Patentansprüche für den Vertragsstaat: AT**

1. Ein Verfahren für die Herstellung einer Verbindung der Strukturformel I:

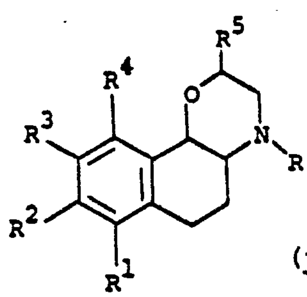

I

(trans)

mit positiver, negativer oder Null betragender optischer Aktivität, und deren Salzen, worin:

R a) Wasserstoff,
b) $C_{1-4}$-Alkyl,
c) $C_{2-5}$-Alkenyl oder
d) Phenyl-$C_{1-4}$-alkyl ist; und

$R^1$, $R^2$, $R^3$ und $R^4$ unabhängig voneinander:
a) Wasserstoff,
b) $C_{1-4}$-Alkyl,
c) Halogen,
d) $OR^6$ sind, wobei $R^6$
1) Wasserstoff

2) $C_{1-3}$-Alkyl,
3) Phenyl-$C_{1-3}$-alkyl oder,
4) $-\underset{\underset{O}{\|}}{C}-R^7$ ist, wobei $R^7$

i) $C_{1-6}$-Alkyl,
ii) $C_{3-6}$-Cycloalkyl,
iii) Phenyl-$C_{1-3}$-alkyl, wobei die Phenylgruppe unsubstituiert oder durch einen oder mehrere Halogen-, $C_{1-3}$-Alkyl- oder $C_{1-3}$-Alkoxyreste substituiert ist,
iv) Pyridyl-$C_{1-3}$-alkyl oder Furyl-$C_{1-3}$-alkyl,
v) Phenyl, das entweder unsubstituiert oder durch einen oder mehrere Halogen-, $C_{1-3}$-Alkyl- oder $C_{1-3}$-Alkoxyreste substituiert ist,
vi) Pyridyl oder Furyl, oder
vii) $-NR^8R^9$ ist, wobei $R^8$ und $R^9$ unabhängig voneinander Wasserstoff oder $C_{1-3}$-Alkyl sind, oder $R^8$ und $R^9$ miteinander verbunden sein können, um mit dem Stickstoff, an den sie gebunden sind, einen Heterocyclus zu bilden, wobei der Heterocyclus aus Piperidinyl, Morpholinyl, Piperazinyl und N—$C_{1-3}$-Alkylpiperazinyl ausgewählt ist, oder

e) zwei benachbarte, aus $R^1$, $R^2$, $R^3$ und $R^4$ ausgewählte Gruppen gemeinsam eine Alkylendioxygruppe bilden, und

$R^5$ Wasserstoff, $C_{1-3}$-Alkyl oder Phenyl ist, welches die Reduktion einer Verbindung der Strukturformel II:

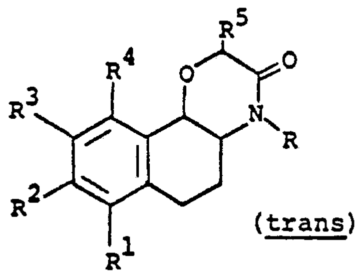

(trans)

II

mit positiver, negativer oder Null betragender optischer Aktivität umfaßt, an die sich gewünschtenfalls, falls R Wasserstoff ist, eine Alkylierung oder Alkenylierung zur Bildung der Verbindung, worin R $C_{1-4}$-Alkyl, $C_{2-5}$-Alkenyl oder Phenyl-$C_{1-4}$-alkyl ist, anschließt, und/oder anschließend, falls erwünscht, falls einer oder mehrere der Reste $R^1$, $R^2$, $R^3$ und $R^4$ $C_{1-3}$-Alkoxy oder Phenyl-$C_{1-3}$-alkoxy sind, oder zwei benachbarte, aus $R^1$, $R^2$, $R^3$ und $R^4$ ausgewählte Gruppen zusammengenommen eine Alkylendioxygruppe sind, eine Entetherung zur Bildung der Verbindung, worin einer oder mehrere der Reste $R^1$, $R^2$, $R^3$ und $R^4$ Hydroxy sind, vorgenommen wird; und/oder anschließend, falls erwünscht, eine Acylierung oder Carbamylierung mit einem Anhydrid der Formel $(R^7CO)_2O$ oder einem Säurechlorid der Struktur $R^7COCl$ zur Bildung der Verbindung, worin einer oder mehrere der Reste $R^1$, $R^2$, $R^3$ und $R^4$

$$R^7\overset{\overset{O}{\|}}{C}-O-$$

sind, vorgenommen wird, und anschließend die Verbindung der Formel I, oder ein Salz davon, isoliert wird.

2. Verfahren nach Patentanspruch 1, dadurch gekennzeichnet, daß Verbindungen der Formel I oder deren Salze hergestellt werden, worin R und $R^5$ die gleiche Bedeutung wie in Anspruch 1 haben, und $R^1$, $R^2$, $R^3$ und $R^4$ unabhängig voneinander:

a) Wasserstoff,
b) $C_{1-4}$-Alkyl,
c) Halogen, oder
d) $OR^6$ sind, wobei $R^6$
1) Wasserstoff,
2) $C_{1-3}$-Alkyl,
3) Phenyl-$C_{1-3}$-alkyl, oder
4) $-\underset{\underset{O}{\|}}{C}-R^7$ ist, wobei $R^7$ wie unter i) bis vi) in Anspruch 1 definiert ist, oder

vii) $-NR^8R^9$ ist, wobei $R^8$ und $R^9$ unabhängig voneinander Wasserstoff oder $C_{1-3}$-Alkyl sind, oder $R^8$ und $R^9$ mit dem Stickstoff, an den sie gebunden sind, miteinander unter Bildung eines Heterocyclus verbunden sein können, der aus Piperidinyl, Morpholinyl, Piperazinyl und N—$C_{1-3}$-Alkyl-piperazinyl ausgewählt ist, oder

e) zwei benachbarte, aus $R^1$, $R^2$, $R^3$ und $R^4$ ausgewählte Gruppen zusammengenommen eine Methylendioxygruppe bilden.

3. Verfahren nach Patentanspruch 1 oder 2, dadurch gekennzeichnet, daß Verbindungen der Strukturformel Ia:

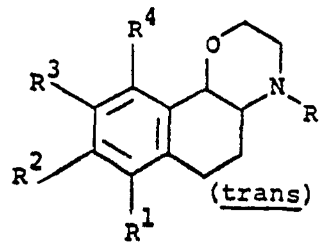

Ia

mit positiver, negativer oder Null betragender optischer Aktivität, oder deren Salze hergestellt werden, worin

R die gleiche Bedeutung wie für Formel I angegeben hat, und $R^1$, $R^2$, $R^3$ und $R^4$ unabhängig voneinander:

a) Wasserstoff,
b) $C_{-1-4}$-Alkyl,
c) Halogen, oder
d) $OR^6$ sind, wobei $R^6$
 1) Wasserstoff,
 2) $C_{1-3}$-Alkyl,
 3) Phenyl-$C_{1-3}$-alkyl oder
 4) $-COR^7$ ist, oder
e) zwei benachbarte, aus $R^1$, $R^2$, $R^3$ und $R^4$ ausgewählte Gruppen miteinander eine Methylendioxygruppe bilden,

und vorzugsweise Verbindungen der Formel Ia hergestellt werden, worin
R n-Propyl ist,
$R^3$ Hydroxy oder Methoxy ist, und
$R^1$, $R^2$ und $R^4$ Wasserstoff sind,
in welchem Verfahren eine Verbindung der Strukturformel IIa

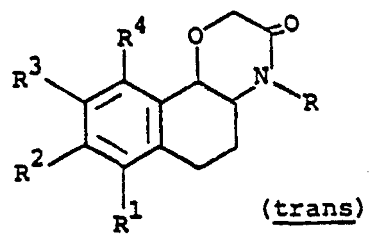

IIa

mit positiver, negativer oder Null betragender optischer Aktivität reduziert wird, worauf gewünschtenfalls, falls R Wasserstoff ist, eine Alkylierung unter Bildung der Verbindung erfolgt, worin R $C_{1-4}$-Alkyl $C_{2-5}$-Alkenyl oder Phenyl-$C_{1-4}$-alkyl ist, und/oder worauf gewünschtenfalls, falls einer oder mehrere der Reste $R^1$, $R^2$, $R^3$ und $R^4$ $C_{1-3}$-Alkoxy oder Phenyl-$C_{1-3}$-alkoxy sind, oder R-Gruppen zusammengenommen Methylendioxy sind, eine Entetherung vorgenommen wird, um die Verbindung zu bilden, worin einer oder mehrere der Reste $R^1$, $R^2$, $R^3$ und $R^4$ Hydroxy sind, und wobei anschließend die Verbindungen der Formel Ia, oder ein Salz davon, isoliert werden.

4. Ein Verfahren zur Herstellung einer Verbindung der Strukturformel Ib

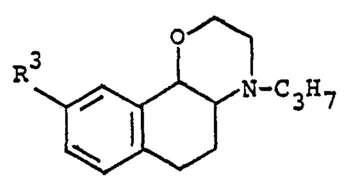

Ib

mit Null betragender oder positiver optischer Aktivität, worin $R^3$ $-OH$ oder $-OCH_3$ ist, oder eines Salzes der Verbindungen der Formel Ib, welches umfaßt:

(a) die Reduktion der 4-Propionylgruppe einer Verbindung der Strukturformel:

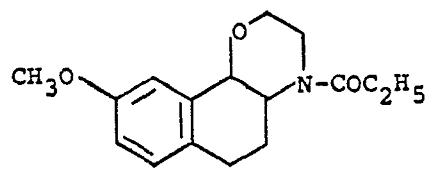

mit Null betragender oder positiver optischer Aktivität, anschließend gewünschtenfalls die optische Trennung in das positive Enantiomer, und anschließend gewünschtenfalls die Entmethylierung unter Bildung der Verbindung, worin $R^3$ —OH ist;

(b) die reduktive Alkylierung einer 4-Wasserstoffverbindung der Strukturformel:

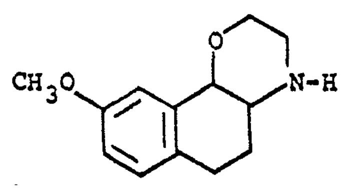

mit Null betragender oder positiver optischer Aktivität, durch Behandlung mit Propionaldehyd und einem Reduktionsmittel, anschließend gewünschtenfalls die optische Trennung in das positive Enantiomer, und anschließend gewünschtenfalls die Entmethylierung unter Bildung der Verbindung, worin $R^3$ OH ist;

(c) die Doppelkondensations-Tetrahydrooxazinringbildung durch Behandlung einer Verbindung der Strukturformel:

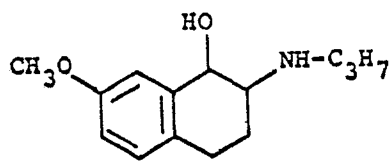

mit Null betragender oder positiver optischer Aktivität, mit einer Verbindung der Strukturformel:

$$X'—CH_2CH_2—X^2$$

worin X' und $X^2$ gleich oder verschieden sind und Brom, Iod, mehrkerniges, aromatisches Sulfonyloxy oder $C_{1-3}$-Alkansulfonyloxy bedeuten, anschließend gewünschtenfalls die optische Trennung in das positive Enantiomer, und anschließend gewünschtenfalls die Entmethylierung unter Bildung der Verbindung, worin $R^3$ OH ist;

(d) den Schluß des Tetrahydrooxazinrings durch Behandlung einer Verbindung der Strukturformel

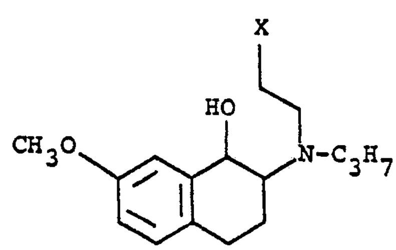

mit Null betragender oder positiver optischer Aktivität, worin X Brom, Iod, mehrkerniges, aromatisches Arylsulfonyloxy, $C_{1-3}$-Alkansulfonyloxy oder Hydroxy ist, mit einem Kondensationsmittel, anschließend gewünschtenfalls die optische Trennung in das positive Enantiomer, und anschließend gewünschtenfalls die Entmethylierung unter Bildung der Verbindung, worin $R^3$ OH ist;

(e) die Reduktion einer 4-Allylgruppe durch Behandlung einer Verbindung der Strukturformel:

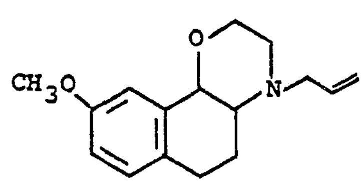

mit Null betragender oder positiver optischer Aktivität, mit einem Reduktionsmittel, anschließend gewünschtenfalls die optische Trennung in das positive Enantiomer, und anschließend gewünschtenfalls die Entmethylierung unter Bildung der Verbindung, worin $R^3$ OH ist;

(f) die Methylierung einer Verbindung der Strukturformel:

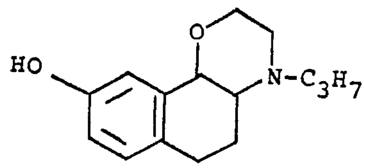

mit Null betragender oder positiver optischer Aktivität, durch Behandlung mit einem Methylierungsmittel unter Bildung der Verbindung, worin $R^3$ —$OCH_3$ ist, und anschließend gewünschtenfalls die Trennung in das positive Enantiomer;

(g) die Reduktion einer Verbindung der Strukturformel:

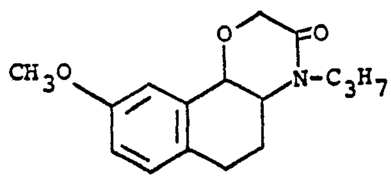

mit Null betragender oder positiver optischer Drehung durch Behandlung mit einem Reduktionsmittel, anschließend gewünschtenfalls die optische Trennung in das positive Enantiomer, und anschließend gewünschtenfalls die Entmethylierung unter Bildung der Verbindung, worin $R^3$ OH ist; und weiterhin die Isolierung der Verbindung der Formel Ib oder des Salzes derselben.

5. Verfahren zur Herstellung einer pharmazeutischen Zusammensetzung für die Behandlung von Parkinsonismus, Depressionen oder Hypertension, dadurch gekennzeichnet, daß eine pharmazeutische Zusammensetzung formuliert wird, welche als pharmazeutisch wirksamen Bestandteil wenigstens eine Verbindung der Strukturformel I:

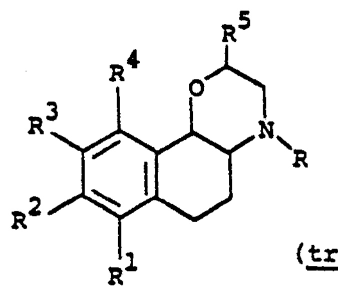

(trans)

I

mit positiver oder Null betragender optischer Aktivität, welche nach dem Verfahren des Anspruchs 1 hergestellt worden ist, und worin: R, $R^1$, $R^2$, $R^3$, $R^4$ und $R^5$ die gleiche Bedeutung wie in Anspruch 1 haben, oder ein pharmazeutisch annehmbares Salz der Verbindungen der Formel I enthält.

6. Verfahren nach Anspruch 5, dadurch gekennzeichnet, daß die pharmazeutische Zusammensetzung unter Verwendung einer Verbindung der Formel I, hergestellt nach dem Verfahren des Patentanspruchs 2, oder eines pharmazeutisch annehmbaren Salzes davon, als Wirkbestandteil, formuliert wird.

7. Verfahren nach Patentanspruch 5, dadurch gekennzeichnet, daß eine pharmazeutische Zusammensetzung formuliert wird, indem als Wirkbestandteil eine Verbindung der Formel Ia, hergestellt nach dem Verfahren des Anspruchs 3, oder ein pharmazeutisch annehmbares Salz davon, verwendet wird.

8. Verfahren nach Anspruch 5, dadurch gekennzeichnet, daß die pharmazeutische Zusammensetzung formuliert wird, indem dieser als Wirkbestandteil eine Verbindung der Formel Ib, hergestellt nach dem Verfahren von Anspruch 5, oder ein pharmazeutisch annehmbares Salz davon, einverleibt wird.

9. Verfahren nach einem der Patentansprüche 5 bis 8, dadurch gekennzeichnet, daß eine pharmazeutische Zusammensetzung hergestellt wird, welche als weitere Komponente ein pharmazeutisches Trägermaterial enthält.

**Revendications pour les Etats contractants: BE CH DE FR GB IT LI LU NL SE**

1. Composés de formule développée I:

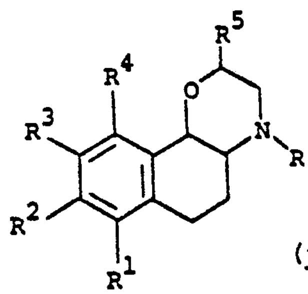

I

(trans)

avec activité optique positive, négative ou nulle, ou leurs sels, où
R est
- a) hydrogène
- b) alcoyle en $C_1$ à $C_4$
- c) alcényle en $C_2$ à $C_5$
- d) phényl-alcoyle en $C_1$ à $C_4$ et

$R^1$, $R^2$, $R^3$ et $R^4$ représentent indépendamment:
- a) hydrogène,
- b) alcoyle en $C_1$ à $C_4$,
- c) halo,
- d) $OR^6$ où $R^6$ est
  - 1) hydrogène,
  - 2) alcoyle en $C_1$ à $C_3$
  - 3) phényl-alcoyle en $C_1$ à $C_3$
  - 4) $—C—R^7$ où $R^7$ est
    ‖
    O
    - i) alcoyle en $C_1$ à $C_6$
    - ii) cycloalcoyle en $C_3$ à $C_6$
    - iii) phényl-alcoyle en $C_1$ à $C_3$ où le groupe phényle est substitué ou non-substitué par un ou plusieurs halos, alcoyle en $C_1$ à $C_3$ ou alcoxy en $C_1$ à $C_3$
    - iv) pyridyl-alcoyle en $C_1$ à $C_3$ ou furyl-alcoyle en $C_1$ à $C_3$
    - v) phényle, non-substitué ou substitué par un ou plusieurs halo, alcoyle en $C_1$ à $C_3$ ou alcoxy en $C_1$ à $C_3$,
    - vi) pyridyle ou furyle et
    - vii) $—NR^8R^9$, où $R^8$ et $R^9$ représentent indépendamment un hydrogène ou un alcoyle en $C_1$ à $C_3$, ou $R^8$ et $R^9$ peuvent être rassemblés pour former un noyau hétérocyclique pipéridinyle, morpholinyle, pipérazinyle ou N-alcoyle en $C_1$ à $C_3$-pipérazinyle avec l'azote auquel ils sont attachés,
- e) deux groupes adjacents choisis entre $R^1$, $R^2$, $R^3$ et $R^4$ forment ensemble un groupe alcoylène-dioxy et

$R^5$ est un hydrogène, un alcoyle en $C_1$ à $C_3$ ou un phényl.

2. Composé de formule I ou un de ses sels où:
R et $R^5$ ont la même signification que dans la revendication 1 et
$R^1$, $R^2$, $R^3$ et $R^4$ représentent indépendamment:
- a) hydrogène,
- b) alcoyle en $C_1$ à $C_4$
- c) halo
- d) $OR^6$ où $R^6$ est
  - 1) hydrogène
  - 2) alcoyle en $C_1$ à $C_3$
  - 3) phényl-alcoyle en $C_1$ à $C_3$
  - 4) $—C—R^7$ où $R^7$ est tel que défini en i) à vi) dans la revendication 1 ou
    ‖
    O
    - vii) $—NR^8R^9$, où $R^8$ et $R^9$ représentent indépendamment un hydrogène ou un alcoyle en $C_1$ à $C_3$, ou $R^8$ et $R^9$ peuvent être réunis pour former avec l'azote auquel ils sont attachés un hétérocycle choisi entre pipéridinyle, morpholinyle, pipérazinyle et N-alcoyle en $C_1$ à $C_3$-pipérazinyle
- e) deux groupes adjacents choisis entre $R^1$, $R^2$, $R^3$ et $R^4$ forment ensemble un groupe méthylène-dioxy.

3. Composé des revendications 1 ou 2 ayant la formule développée la

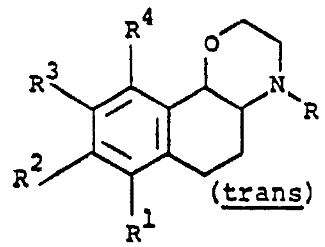

Ia

avec activité optique positive, négative ou nulle, ou ses sels où:
R a la même signification que dans la formule I de la revendication 1 et $R^1$, $R^2$, $R^3$ et $R^4$ représentent indépendamment:

a) hydrogène,
b) alcoyle en $C_1$ à $C_4$
c) halo
d) $OR^6$ ou $R^6$ représente
    1) hydrogène
    2) alcoyle en $C_1$ à $C_3$
    3) phényle-alcoyle en $C_1$ à $C_3$
    4) —CO—$R^7$ ou
e) deux groupes adjacents choisis entre $R^1$, $R^2$, $R^3$ et $R^4$ forment ensemble un groupe méthylène-dioxy et de préférence un composé de formule Ia où:

R est un n-propyle,
$R^3$ est un hydroxy ou un méthoxy et
$R^1$, $R^2$ et $R^4$ représentent un hydrogène.

4. Procédé de préparation d'un composé de formule développée Ia:

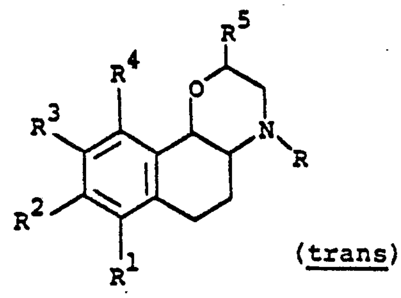

I

selon la revendication 1 avec activité positive, négative ou nulle, ou les sels du composé de formule I où R, $R^1$, $R^2$, $R^3$, $R^4$ et $R^5$ ont la même signification que dans la revendication 1, caractérisé en ce qu'on réduit des composés de formule II:

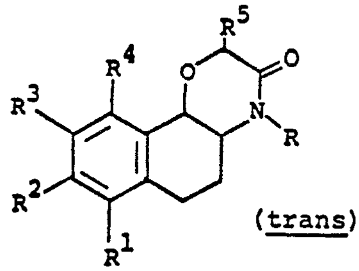

II

à activité optique positive, négative ou nulle, où les radicaux R, $R^1$, $R^2$, $R^3$, $R^4$ et $R^5$ ont la même signification que dans la formule I, puis, si on le désire lorsque R est un hydrogène, on procède par alcoylation ou alcénylation pour fournir le composé où R est un alcoyle en $C_1$ à $C_4$, un alcényle en $C_2$ à $C_5$ ou un phénylalcoyle en $C_1$à $C_4$ et/ou ensuite, si on le desire, lorsque un ou plusieurs des radicaux $R^1$, $R^2$, $R^3$ et $R^4$ est un alcoxy en $C_1$ à $C_3$, un phénylalcoxy en $C_1$ à $C_3$, ou deux d'entre eux sont des groupes adjacents choisis entre $R^1$, $R^2$, $R^3$ et $R^4$ et représentent ensemble un groupe alcoylène-dioxy, on procède par désétherification pour fournir le composé où un ou plusieurs des radicaux $R^1$, $R^2$, $R^3$ et $R^4$ est un hydroxy; et/ou ensuite, si on le désire, on procède par acylation ou carbamylation avec un anhydride de formule $(R^7CO)_2O$ ou un chlorure

d'acide de structure $R^7COCl$ pour produire le composé où ou plusieurs des radicaux $R^1$, $R^2$, $R^3$ et $R^4$ représente

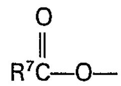

et en ce que qu'ensuite on isole les composés de formule I ou leurs sels.

5. Procédé selon la revendication 4 ou l'on prépare les composés de la revendication 2 ou leurs sels.

6. Procédé selon les revendications 4 ou 5, caractérisé en ce qu'on prépare les composés de formule développée

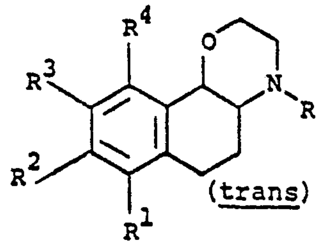

Ia

selon la revendication 3 à activité optique positive, négative ou nulle, ou leurs sels, où:
R, $R^1$, $R^2$, $R^3$ et $R^4$ ont la même signification que dans la revendication 3, caractérisé en ce qu'on réduit les composés de formule développée IIa

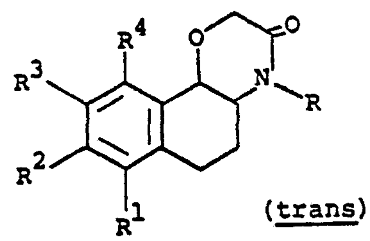

IIa

à activité optique positive, négative ou nulle, où R, $R^1$, $R^2$, $R^3$ et $R^4$ ont la même signification que dans la formule Ia, puis, si on le désire lorsque R est un hydrogène, on procède par alcoylation pour fournir le composé où R est un alcoyle en $C_1$ à $C_4$, un alcényle en $C_1$ à $C_5$, ou un phényl-alcoyle en $C_1$ à $C_4$, et/ou ensuite, si on le désire, lorsque un ou plusieurs des radicaux $R^1$, $R^2$, $R^3$ et $R^4$ est un alcoxy en $C_1$ à $C_3$, un phényl-alcoxy en $C_1$ à $C_3$ ou des groupes R pris ensemble représentent un méthylène dioxy, on procède par désétherification pour fournir le composé où un ou plusieurs des radicaux $R^1$, $R^2$, $R^3$ et $R^4$ est un hydroxy et où ensuite on isole les composés de formule Ia ou un de leurs sels.

7. Procédé de préparation d'un composé de formule développée Ib

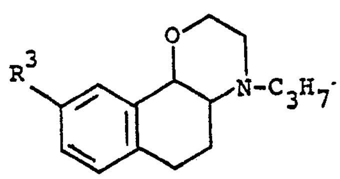

Ib

ou d'un sel du composé de formule Ib, où $R^3$ est —OH ou —$OCH_3$, caractérisé en ce que le dit composé comprend

a) La réduction du groupe 4-propionyle d'un composé de formule développée

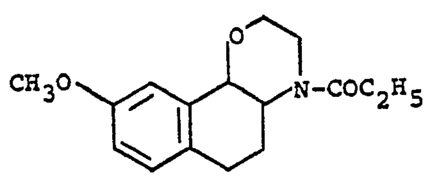

à activité optique nulle ou positive suivie, si on le désire, par un dédoublement optique pour donner l'énantiomère positif puis, si on le désire, par une déméthylation pour produire le composé où $R^3$ représente —OH;

b) L'alcoylation réductrice d'un composé 4-hydrogène de formule développée

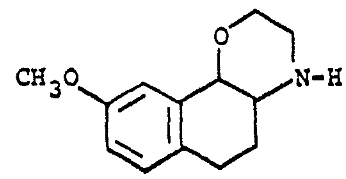

à activité optique nulle ou positive par traitement avec du propionaldéhyde et un agent réducteur suivi si on le désire par un dédoublement optique pour donner l'énantiomère positif suivi, si on le désire, par une déméthylation pour produire le composé où $R^3$ représente —OH;

c) Une formation de noyau tétrahydro-oxazine par double condensation par traitement d'un composé de formule développée

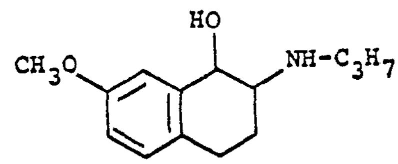

à activité optique nulle ou positive avec un composé de formule développée:

$$X'—CH_2CH_2—X^2$$

où X' et $X^2$ sont semblables ou différents et représentent bromo, iodo, sulfonyloxy aromatique benzénoïde, ou alcanesulfonyloxy en $C_1$ à $C_3$, suivi si on le désire par un dédoublement optique pour donner l'énantiomère positif, suivi si on le désire par une déméthylation pour produire le composé où $R^3$ représente OH;

d) La fermeture du noyau tétrahydro-oxazine par traitement d'un composé de formule développée

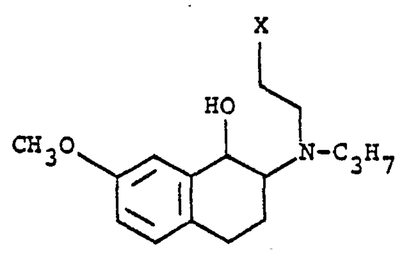

à activité optique nulle ou positive où X est un bromo, iodo, arylsulfonyloxy benzénoïde, alcanesulfonyloxy en $C_1$ à $C_3$ ou hydroxy, avec un agent de condensation, suivi si on le désire par un dédoublement optique pour donner l'énantiomère positif, suivi si on le désire par une déméthylation pour produire le composé où $R^3$ représente OH;

e) La réduction d'un groupe 4-allyle par traitement d'un composé de formule développée

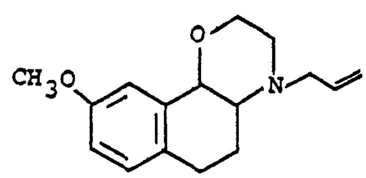

à activité optique nulle ou positive avec un agent réducteur suivi si on le désire par un dédoublement optique pour donner l'énantiomère positif, suivi si on le désire par une déméthylation pour produire le composé où $R^3$ représente OH;

f) Une méthylation d'un composé de formule développée

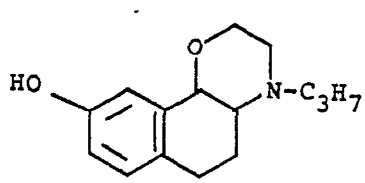

à activité optique nulle ou positive par traitement avec un agent méthylant, pour produire le composé où $R^3$ représente —$OCH_3$ suivi si on le désire par un dédoublement pour donner l'énantiomère positif;

g) Une réduction d'un composé de formule développée

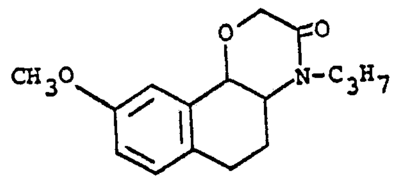

à rotation optique nulle ou positive par traitement avec un agent réducteur, suivi si on le désire par un dédoublement optique pour donner l'énantiomère positif, suivi si on le désire par une déméthylation pour produire le composé où $R^3$ représente OH;

puis l'isolement du composé de formule Ib ou de son sel.

8. Formulation pharmaceutique pour le traitement de la maladie de Parkinson, des dépressions ou de l'hypertension comprenant comme ingrédient actif un composé de formule développée I:

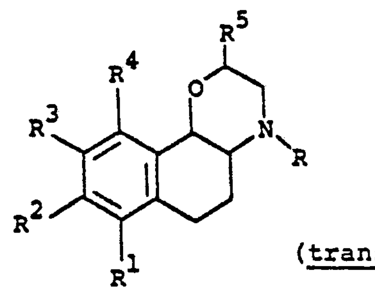

I

(trans)

à activité optique positive ou nulle, ou un de ses sels pharmaceutiquement acceptables, où R, $R^1$, $R^2$, $R^3$, $R^4$ et $R^5$ ont la même signification que dans la revendication 1.

9. Formulation pharmaceutique selon la revendication 8, caractérisé en ce qu'elle comprend comme ingrédient actif un composé de formule I selon la revendication 2 ou un sel pharmaceutiquement acceptable du dit composé de formule I et, en outre, une matière pharmaceutique support.

10. Formulation pharmaceutique selon les revendications 8 ou 9, caractérisée en ce qu'elle comprend comme composé pharmaceutiquement efficace un composé de formule Ia selon la revendication 3 ou un de ses sels pharmaceutiquement acceptable et, en outre, un support pharmaceutique.

**Revendications pour l'Etat contractant: AT**

1. Procédé de préparation d'un composé de formule I:

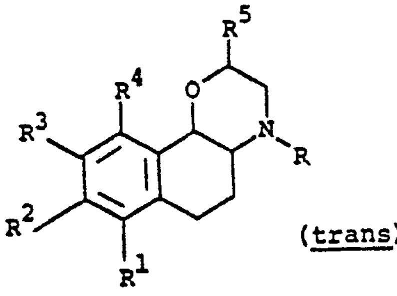

(trans)

à activité optique positive, négative ou nulle, et de ses sels, où

R représente

a) hydrogène

b) alcoyle en $C_1$ à $C_4$

c) alcényle en $C_2$ à $C_5$

d) phényl-alcoyle en $C_1$ à $C_4$; et

$R^1$, $R^2$, $R^3$, et $R^4$ représentent indépendamment:

a) hydrogène

b) alcoyle en $C_1$ à $C_4$

c) halo

d) $OR^6$ où $R^6$ représente
1) hydrogène
2) alcoyle en $C_1$ à $C_3$
3) phényl-alcoyle en $C_1$ à $C_3$
4) $-\underset{\underset{O}{\|}}{C}-R^7$ où $R^7$ est

i) alcoyle en $C_1$ à $C_6$
ii) cycloalcoyle en $C_3$ à $C_6$
iii) phényl-alcoyle en $C_1$ à $C_3$, où le groupe phényle est non-substitué ou substitué par un ou plusieurs halo, alcoyle en $C_1$ à $C_3$, ou alcoxy en $C_1$ à $C_3$
iv) pyridyl-alcoyle en $C_1$ à $C_3$ ou furyl-alcoyle en $C_1$ à $C_3$
v) phényle, non-substitué ou substitué par un ou plusieurs halos, alcoyle en $C_1$ à $C_3$ ou alcoxy en $C_1$ à $C_3$,
vi) pyridyle ou furyle et
vii) $-NR^8R^9$, où $R^8$ et $R^9$ représentent indépendamment un hydrogène ou un alcoyle en $C_1$ à $C_3$, ou $R^8$ et $R^9$ peuvent être réunis pour former un hétérocycle avec l'azote auquel ils sont attachés, l'hétérocycle étant choisi entre pipéridinyle, morpholinyle, pipérazinyle et N-alcoyle en $C_1$ à $C_3$ pipérazinyle,

e) deux groupes adjacents choisis entre $R^1$, $R^2$, $R^3$ et $R^4$ forment ensemble un groupe alcoylène-dioxy et $R^5$ est un hydrogène, un alcoyle en $C_1$ à $C_3$ ou un phényle, comprenant la réduction d'un composé de formule développée II:

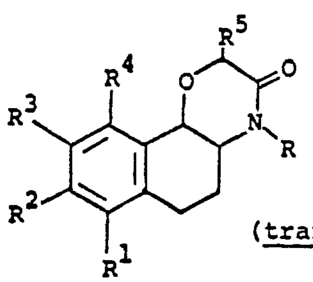
(trans)

(II)

à activité optique positive, négative ou nulle, suivie si on le désire lorsque R est un hydrogène, par une alcoylation ou une alcénylation pour former le composé où R représente un alcoyle en $C_1$ à $C_4$, alcényle en $C_2$ à $C_5$ ou phényle-alcoyle en $C_1$ à $C_4$ et/ou suivie si on le désire lorsque un ou plusieurs des radicaux $R^1$, $R^2$, $R^3$ et $R^4$ est un alcoxy en $C_1$ à $C_3$, phényle-alcoxy en $C_1$ à $C_3$, ou deux d'entre eux sont des groupes adjacents choisis entre $R^1$, $R^2$, $R^3$ et $R^4$ et forment ensemble un groupe alcoylène-dioxy, par déséthérification pour fournir le composé où un ou plusieurs des radicaux $R^1$, $R^2$, $R^3$ et $R^4$ est un hydroxy; et/ou suivie si on le désire par une acylation ou carbamylation avec un anhydride de formule $(R^7CO)_2O$ ou un chlorure d'acide de structure $R^7COCl$ pour produire le composé où un plusieurs des radicaux $R^1$, $R^2$, $R^3$ et $R^4$ représentent

$$R^7\overset{\overset{O}{\|}}{C}-O-$$

et, l'isolement du composé de formule I ou d'un de ses sels.

2. Procédé selon la revendication 1 caractérisé en ce qu'on prépare des composés de formule I ou leurs sels où R et $R^5$ ont la même signification que dans la revendication 1 et $R^1$, $R^2$, $R^3$ et $R^4$ représentent indépendamment:
a) hydrogène
b) alcoyle en $C_1$ à $C_4$
c) halo
d) $OR^6$ où $R^6$ représente
1) hydrogène
2) alcoyle en $C_1$ à $C_3$
3) phényl-alcoyle en $C_1$ à $C_3$
4) $-\underset{\underset{O}{\|}}{C}-R^7$ où $R^7$ est tel que défini en i) à vi) dans la revendication 1 où

vii) $-NR^8R^9$, où $R^8$ et $R^9$ représentent indépendamment un hydrogène ou un alcoyle en $C_1$ à $C_3$, ou $R^8$ et $R^9$ peuvent être réunis pour former avec l'azote auquel ils sont attachés un hétérocycle choisi entre pipéridinyle, morpholinyle, pipérazinyle et N-alcoyle en $C_1$ à $C_3$-pipérazinyle

e) deux groupes adjacents choisis entre $R^1$, $R^2$, $R^3$ et $R^4$ forment ensemble un groupe méthylène-dioxy.

3. Procédé selon la revendication 1 ou 2, caractérisé en ce qu'on prépare des composés de formule développée Ia:

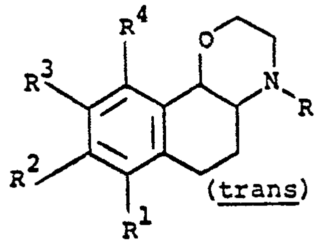

Ia

à activité optique positive, négative ou nulle, ou leurs sels où R a la même signification que dans la formule I et $R^1$, $R^2$, $R^3$ et $R^4$ représentent indépendamment:

a) hydrogène
b) alcoyle en $C_1$ à $C_4$
c) halo
d) $OR^6$ où $R^6$ représente
  1) hydrogène
  2) alcoyle en $C_1$ à $C_3$
  3) phényl-alcoyle en $C_1$ à $C_3$
  4) $—COR^7$ ou
e) deux groupes adjacents choisis entre $R^1$, $R^2$, $R^3$ et $R^4$ forment ensemble un groupe méthylène-dioxy
et de préférence on prépare des composés de formule Ia où
R représente un n-propyle
$R^3$ représente un hydroxy ou un méthoxy et
$R^1$, $R^2$ et $R^3$ sont des hydrogènes,
dans lequel procédé on réduit un composé de formule développée IIa

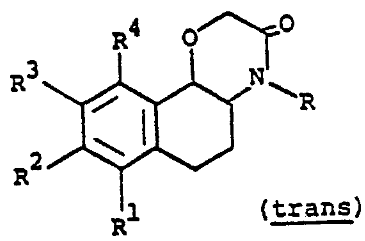

IIa

à activité optique positive, négative ou nulle, puis, si on le désire lorsque R est un hydrogène, on procède à une alcoylation pour former le composé où R est un alcoyle en $C_1$ à $C_4$, un alcényle en $C_2$ à $C_5$, ou un phényl-alcoyle en $C_1$ à $C_4$, et/ou, si on le désire, en ce que lorsque un ou plusieurs des radicaux $R^1$, $R^2$, $R^3$ et $R^4$ est un alcoxy en $C_1$ à $C_3$, un phényl-alcoxy en $C_1$ à $C_3$, ou les groupes R pris ensemble représentent un méthylène-dioxy, on procède par déséthérification pour fournir le composé où un ou plusieurs des radicaux $R^1$, $R^2$, $R^3$ et $R^4$ est un hydroxy et où ensuite on isole les composés de formule Ia ou un de leurs sels.

4. Procédé de préparation d'un composé de formule développée Ib:

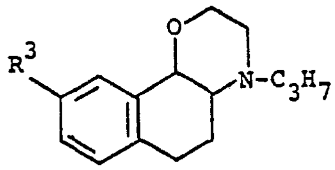

Ib

à activité optique nulle ou positive où $R^3$ est $—OH$ ou $—OCH_3$ ou un sel des composés de formule Ib comprenant:

a) réduction du groupe 4-propionyle d'un composé de formule développée:

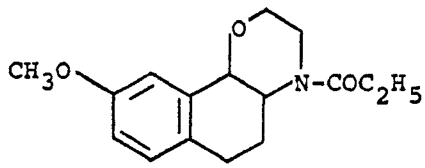

à activité optique nulle ou positive suivie, si on le désire par un déboulement optique pour donner l'énantiomère positif suivi, si on le désire par une déméthylation pour produire le composé où $R^3$ est —OH;

b) alcoylation réductrice d'un composé 4-hydrogène de formule

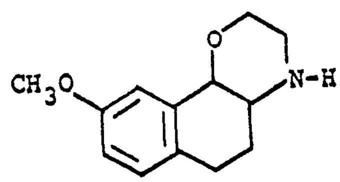

à activité optique nulle ou positive par traitement avec du propionaldéhyde et un agent réducteur suivi si on le désire par un dédoublement optique pour donner l'énantiomère positif, suivi si on le désire par une déméthylation pour produire le composé où $R^3$ est OH;

c) formation d'un noyau tétrahydro-oxazine par double condensation par traitement d'un composé de formule développée:

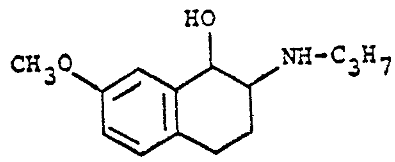

à activité optique nulle ou positive avec un composé de formule développée:

$$X'—CH_2CH_2—X^2$$

où X' et $X^2$ sont semblables ou différents, représentent un bromo, iodo, sulfonyloxy aromatique benzénoïde ou alcanesulfonyloxy en $C_1$ à $C_3$, suivi si on le désire par un dédoublement optique pour donner l'énantiomère positif, suivi si on le désire par une déméthylation pour produire le composé où $R^3$ est OH;

d) fermeture du noyau tétrahydro-oxazine par traitement d'un composé de formule développée;

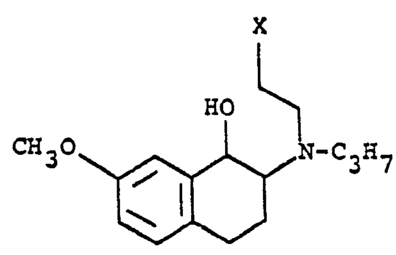

à activité optique nulle ou positive où X est un bromo, iodo, arylsulonyloxy benzénoïde, alcanesulfonyloxy en $C_1$ à $C_3$ ou hydroxy, avec un agent de condensation, suivi si on le désire par un dédoublement optique pour donner l'énantiomère positif, suivi si on le désire par une déméthylation pour produire le composé où $R^3$ est OH;

e) réduction d'un groupe 4-allyle par traitement d'un composé de formule développée

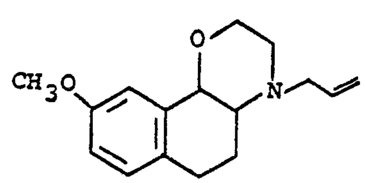

à activité optique nulle ou positive avec un agent réducteur suivi si on le désire par un dédoublement optique pour donner l'énantiomère positif, suivi si on le désire par une déméthylation pour produire le composé où $R^3$ est OH;

f) méthylation d'un composé de formule développée

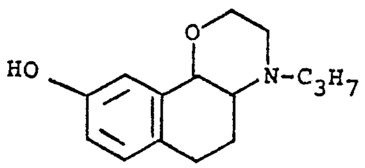

à activité optique nulle ou positive par traitement avec un agent méthylant, pour produire le composé où $R^3$ est $—OCH_3$ suivi si on le désire par un dédoublement pour donner l'énantiomère positif;

g) réduction d'un composé de formule développée

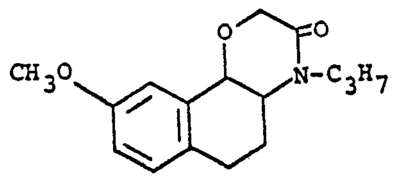

à rotation optique nulle ou positive par traitement avec un agent réducteur, suivi si on le désire par un dédoublement optique pour donner l'énantiomère positif, suivi si on le désire par une déméthylation pour produire le composé où $R^3$ est OH;

et ensuite isolement du composé de formule Ib ou de son sel.

5. Procédé de préparation d'une composition pharmaceutique pour le traitement de la maladie de Parkinson, des dépressions ou de l'hypertension, caractérisé en ce qu'on formule une composition pharmaceutique contenant comme ingrédient pharmaceutiquement actif au moins un composé de formule développée I:

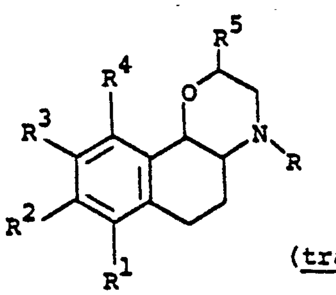

I

(trans)

à activité optique positive ou nulle, préparé selon le procédé de la revendication 1, où R, $R^1$, $R^2$, $R^3$, $R^4$ et $R^5$ ont la même signification que dans la revendication 1, ou un sel pharmaceutiquement acceptable des composés de formule I.

6. Procédé selon la revendication 5 caractérisé en ce qu'on formule la composition pharmaceutique en utilisant comme ingrédient actif un composé de formule I préparé selon le procédé de la revendication 2 ou un de ses sels pharmaceutiquement acceptables.

7. Procédé selon la revendication 5 caractérisé en ce qu'on prépare une composition pharmaceutique en formulant comme ingrédient actif un composé de formule Ia préparé selon le procédé de la revendication 3 ou un de ses sels pharmaceutiquement acceptables.

8. Procédé selon la revendication 5 caractérisé en ce qu'on formule la composition pharmaceutique en y incorporant comme ingrédient actif un composé de formule Ib préparé selon le procédé de la revendication 5 ou un de ses sels pharmaceutiquement acceptables.

9. Procédé selon l'une des revendications 5—8 caractérisé en ce qu'on prépare une composition pharmaceutique contenant comme autre composant un support pharmaceutique.